# EUROPEAN PATENT SPECIFICATION

(11) **EP 1 602 404 B1**
(45) Date of publication and mention of the grant of the patent: **14.01.2009**
(21) Application number: 04717864.5
(22) Date of filing: 05.03.2004
(51) Int. Cl.: B01J 19/12, A61K 9/14, A61K 9/08, A61K 31/573, C07J 7/00, A61J 3/02

(54) **MEDICAMENT IN FINE PARTICLE FORM, METHOD AND DEVICE FOR PREPARATION THEREOF, AND AGENT FOR PARENTERAL INJECTION AND METHOD FOR PRODUCTION THEREOF**
MEDIKAMENT IN FORM FEINER TEILCHEN, VERFAHREN UND VORRICHTUNG ZU SEINER HERSTELLUNG UND MITTEL ZUR PARENTERALEN INJEKTION UND HERSTELLUNGSVERFAHREN DAFÜR
MEDICAMENT EN FORME DE PARTICULES FINES, SON PROCEDE ET DISPOSITIF DE PREPARATION, ET AGENT D'INJECTION PARENTERALE ET SON PROCEDE DE PRODUCTION

(30) Priority: 07.03.2003 JP 2003062452; 16.06.2003 JP 2003171051
(43) Date of publication of application: 07.12.2005
(73) Proprietor: HAMAMATSU PHOTONICS K.K., Hamamatsu-shi, Shizuoka-ken 435-8558 (JP)
(72) Inventor: KAWAKAMI, Tomonori, Hamamatsu-shi, Shizuoka 4358558 (JP); HIRAMATSU, Mitsuo, Hamamatsu-shi, Shizuoka 4358558 (JP); SATOZONO, Hiroshi, Hamamatsu-shi, Shizuoka 4358558 (JP); TAKAGI, Tokio, Hamamatsu-shi, Shizuoka 4358558 (JP)
(74) Representative: Frost, Alex John
(86) International application number: PCT/JP2004/002909
(87) International publication number: WO 2004/080586

(56) References cited:
- JP-A- 04 063 203
- JP-A- 2001 113 159
- JP-A- 2001 113 159
- JP-A- 2003 025 299
- US-A- 3 600 291

## Description

### Technical Field

The present invention relates to fine particles, method and apparatus for manufacturing the fine particles, injectable agents and a method for manufacturing the injectable agents, specifically, relates to fine particles of an organic compound, method and apparatus for manufacturing the fine particles, injectable agents and a method for manufacturing the injectable agents.

### Background Art

There is an extreme increase of the surface area to finely pulverize organic compounds into fine particles. Accordingly, there is an advantage in that the reaction between the fine particles thus formed and the surrounding thereof is enhanced, and also properties inherent to the material are likely to appear. Furthermore, when particles are formed of hardly-soluble/insoluble materials, the particles may be kept to be apparently soluble in solvent by finely pulverizing the particles into fine particles (the fine particles are suspended in the solvent, however, it is observed to be apparently soluble because no light scattering occurs).

Therefore, the fine pulverization technique may provide a new material preparing method, and it is expected to be applied to broad technical fields.

Japanese Patent Application Laid-Open No. 2001-113159 discloses one of these fine pulverization methods. This publication discloses a method for generating fine particles of organic compounds by irradiating laser beams. According to this method, organic pigment or aromatic condensed polycyclic compounds which have the intermediate properties between inorganic material and organic material and also has a hard and strong molecular structure are used as organic compounds to be finely pulverized. When fine particles are generated, light having wavelengths in a light absorption band of organic compounds is irradiated to the organic compound to generate fine particles.

### Disclosure of the Invention

If the fine pulverization technique described above is used, a new material preparing method would be provided, and it is expected that this technique is applicable to broad fields. For example, in the development of drugs, when a newly synthesized material has low solubility to solvents such as water or the like, it is impossible to subject the material concerned to physical and chemical research and searching such as screening or the like, or general drug toxicity, general pharmacology, efficacy pharmacology, biochemical research in preclinical tests of animals such as an ADME test (absorption, distribution, metabolism, excretory test), etc. On the other hand, by finely pulverizing the organic compounds, various research on candidate materials for drug development could be performed.

However, the fine particle generating method described in the above publication has the following problem.

That is, according to the above method, in the case of an organic compound having relatively weak chemical bonds in the molecular structure thereof, fine particles can be generated by irradiating light having wavelengths in the light absorption band thereof, however, at the same time a photochemical reaction of the organic compound is partially induced through an electron excitation state, so that the organic compound is decomposed and impurities are generated in some cases. Particularly when the organic compound is a medicament (medical product) to be administered into the body, such impurities cause a side effect, and may have an adverse effect on the living body. Such a situation must be avoided as much as possible. That is, in the medicine manufacturing field, it is a top priority task to minimize generation of impurities in the medicine manufacturing process such as the treatment of medicines, etc.

The present invention has been implemented to solve the above problem, and has an object to provide a fine particle manufacturing method and apparatus that can manufacture fine particles while sufficiently preventing photochemical reactions in organic compounds, fine particles, and injectable agents and a method for manufacturing the injectable agent.

In order to achieve the above object, the inventors of this application have researched a light irradiation condition for enabling fine pulverization of organic compounds such as medicaments in liquid to be treated while avoiding the occurrence of photochemical reactions in organic compounds, and as a result they have found that the above problem can be solved by irradiating laser beams under a specific light irradiation condition to the organic compounds and completed the present invention.

That is, according to the present invention, a fine particle manufacturing method in which an organic compound in solvent of a treatment target liquid is finely pulverized to manufacture a fine particle of the organic compound, comprises: a preparation step of preparing a treatment target liquid containing an organic compound and solvent mixed with each other; wherein the organic compound is a medicament and a laser beam irradiation step of irradiating a laser beam having a wavelength longer than the light absorption band of the organic compound to the treatment target liquid to finely pulverize the organic compound into a fine particle.

According to this manufacturing method, when the organic compound in the treatment target liquid is irradiated with the laser beam having wavelength longer than the light absorption band of the organic compound, the fine particles of the organic compound concerned can be manufactured while sufficiently preventing photochemical reactions of the organic compound in the treatment target liquid.

In the above manufacturing method, when only a slight part of the organic compound is dissolved in the solvent of the treatment target liquid, that is, it is hardly-soluble in the solvent of the treatment target liquid or insoluble by the solvent in the treatment target liquid, the organic compound can be apparently dissolved in the solvent of the treatment target liquid by finely pulverizing the organic compound through irradiation of laser beams. That is, the organic compound can be kept to a state where the fine particles thereof are contained in the treatment target liquid. Here, "hardly-soluble in the solvent of the treatment target liquid" means that the maximum absorbance of the treatment target liquid is 0.01 or more when the absorbance of the treatment target liquid is measured by setting the optical path length to 1 cm and using a general-purpose spectrophotometer (HITACHI U-3500). When the maximum absorbance is less than 0.01, the organic compound is insoluble in the solvent of the treatment target liquid.

In the fine particle manufacturing method, the irradiation light intensity of the laser beam having wavelength longer than the light absorption band of the organic compound to the treatment target liquid is set to less than the irradiation light intensity at which two-photon absorption occurs in the organic compound.

When a laser beam having the irradiation light intensity at which two-photon absorption occurs in the organic compound is irradiated to the organic compound, there is a tendency that a photochemical reaction occurs in the organic compound although the laser beam having wavelength longer than the light absorption band of the organic compound is used to prevent occurrence of the photochemical reaction. Accordingly, a laser beam having an irradiation light intensity less than the irradiation light intensity at which two-photon absorption occurs is irradiated to the organic compound, whereby the fine particles of the organic compound can be manufactured while more sufficiently preventing occurrence of the photochemical reactions in the organic compound.

In the above manufacturing method, it is preferable that during the irradiation of the laser beam to the treatment target liquid, the absorbance of the organic compound in the treatment target liquid is measured to monitor the fine pulverization state of the organic compound. In this case, the fine pulverization state is monitored, and thus the stop/continuation of the irradiation of the laser beam can be determined in accordance with the fine pulverization state, so that the laser beam irradiation can be prevented from being excessively carried out on the organic compound.

Furthermore, according to the manufacturing method, it is preferable that the irradiation light intensity of the laser beam which is irradiated to a chamber and has a wavelength longer than the light absorption band is varied while measuring the transmission light intensity of the laser beam transmitted through the treatment target liquid in the chamber, thereby determining the irradiation light intensity at which two-photon absorption occurs in the organic compound.

When the irradiation light intensity of the laser beam irradiated to the chamber, for accommodating the treatment target liquid, is varied while measuring the transmission light intensity of the laser beam transmitted through the chamber, two-photon absorption occurs in the organic compound at some irradiation light intensity. At this time, the transmission light intensity of the laser beam transmitted through the chamber is sharply reduced. Accordingly, the irradiation light intensity at which the two-photon absorption occurs can be easily determined.

In the above manufacturing method, it is preferable that a stabilizer for stably dispersing fine particles manufactured in the treatment target liquid is added to the treatment target liquid before or during the irradiation of the laser beam to the treatment target liquid. In this case, the temporarily-manufactured fine particles are stably dispersed in the treatment target liquid by the stabilizer, and the fine particles can be sufficiently prevented from aggregating, so that the manufacturing efficiency of the fine particles can be enhanced. Here, the stabilizer is preferably a surfactant. In this case, in addition to the enhancement of the manufacturing coefficient of the fine particles, the photochemical reaction in the organic compound can be sufficiently prevented, and the organic compound can be finely pulverized into fine particles by irradiating the laser beam having a wavelength longer than the irradiation wavelength to the organic compound.

The surfactant is useful to enhance the manufacturing efficiency of the fine particles and increase the wavelength of a laser beam to be irradiated, however, it is desired to be removed after the fine particles are manufactured. Therefore, it is preferable that after the surfactant is added to the treatment target liquid as described above, the treatment target liquid is diluted to separate the fine particles from the surfactant, thereby obtaining an aggregating fine particle which is an aggregate of the fine particles. The aggregating fine particles obtained after the fine particles are manufactured are easily treated in a re-dispersing step.

In the fine particle manufacturing method, when the organic compound is a medicament, the photochemical reaction between the medicine and the laser beam can be sufficiently prevented, and thus the fine particles of the organic compound can be manufactured without losing the medicinal benefits thereof. Furthermore, it is preferable that the solvent in the treatment target liquid is water. The surface area of the medicament is increased by the fine pulverization of the medicine, and thus the absorption performance thereof into the tissues of living bodies is enhanced, so that quick-acting fine particles can be obtained. Still furthermore, when only a part of the medicament is dissolved in water, that is, it is hardly-soluble in water, or it is insoluble in water, the medicament can be apparently dissolved in water.

Furthermore, a fine particle manufacturing apparatus according to the present invention in which an organic compound in solvent of the treatment target liquid is finely pulverized to manufacture a fine particle of the organic compound comprises: a chamber for accommodating treatment target liquid containing an organic compound having a predetermined light absorption band and solvent mixed with each other; and a laser beam source for irradiating the treatment target liquid accomodated in the chamber with a laser beam having a wavelength longer than the light absorption band of the organic compound.

According to this fine particle manufacturing apparatus, when a laser beam having a wavelength longer than the light absorption band of the organic compound is irradiated from the laser beam source to the treatment target liquid accommodated in the chamber, the organic compound can be finely pulverized while sufficiently preventing photochemical reactions in the organic compound of the treatment target liquid.

Furthermore, the apparatus is provided with monitoring light absorption band measuring means for measuring the light absorption band of the organic compound in the treatment target liquid to monitor a fine pulverization state of the organic compound. At this time, when the light absorption band of the organic compound is measured by the monitoring light absorption band measuring means to monitor the fine pulverization state, the stop/continuation of the laser beam irradiation can be determined in accordance with the fine-pulverization state, so that excessive laser beam irradiation to the organic compound can be avoided.

It is preferable that the laser beam source is a wavelength-variable laser. In this case, a laser beam having a proper wavelength can be irradiated to the organic compound in the treatment target liquid on the basis of the light absorption band of the organic compound.

It is preferable that the manufacturing apparatus is further provided with irradiation wavelength determining light absorption band measuring means for discharging a part of the treatment target liquid from the chamber and measuring the light absorption band of the organic compound in the treatment target liquid to determine the wavelength of the laser beam irradiated to the organic compound, wherein the irradiation wavelength determining light absorption band measuring means has a separation filter which can separate solid material from the treatment target liquid discharged from the chamber, and the light absorption band of the organic compound in the treatment target liquid from which the solid material is separated by the separation filter is measured.

According to this manufacturing apparatus, even when the light absorption band of the organic compound is unknown, the light absorption band of the organic compound in the treatment target liquid discharged from the chamber can be immediately measured by the irradiation wavelength determining light absorption band measuring means. In accordance with the light absorption band of the organic compound measured by the light absorption band measuring means, the irradiation wavelength of the wavelength-variable laser can be set to be longer than the light absorption band, and the laser beam having the irradiation wavelength thus set can be irradiated to the organic compound.

Furthermore, even when only a part of the organic compound is dissolved in the solvent of the treatment target liquid, that is, it is hardly-soluble in the solvent, the solid material is separated from the treatment target liquid discharged from the chamber by the separation filter. Therefore, in the irradiation wavelength determining light absorption band measuring means, the light absorption band of the organic compound in the solvent of the treatment target liquid which is transmitted through the separation filter can be accurately measured without being scattered by the solid material. When the organic compound is insoluble in the solvent, for example, water, an absorption spectrum is separately measured through a spectrophotometer by using a mixture solvent of water and organic solvent in which the organic compound concerned is soluble, for example, dimethylsulfoxide, thereby knowing the light absorption band of the organic compound, and then a proper laser irradiation wavelength can be determined.

It is preferable that the above manufacturing apparatus is further provided with a transmission light intensity measuring device for measuring the transmission light intensity of a laser beam transmitted through the treatment target liquid in the chamber, and irradiation light intensity adjusting means for adjusting the irradiation light intensity of the laser beam irradiated from the laser beam source to the chamber.

According to this manufacturing apparatus, the laser beam whose wavelength is longer than the longest wavelength of the light absorption band of the organic compound in the treatment target liquid is irradiated from the laser beam source to the treatment target liquid in the chamber, and the transmission light intensity of the laser beam transmitted through the treatment target liquid is measured by the transmission light intensity measuring device. At this time, when the irradiation light intensity of the laser beam is increased by the irradiation light intensity adjusting means, two-photon absorption occurs in the organic compound at some irradiation light intensity. At this time, the transmission light intensity of the laser beam is sharply reduced. Therefore, the irradiation light intensity at which the two-photon absorption occurs can be easily determined.

Here, it is preferable that the chamber more greatly absorbs a laser beam which has a wavelength longer than the light absorption band and has the irradiation light intensity at which two-photon absorption occurs in the organic compound, as compared with a laser beam having an irradiation light intensity at which no two-photon absorption occurs.

In this case, when the irradiation light intensity reaches the irradiation light intensity at which two-photon absorption occurs in the organic compound, the laser beam is greatly absorbed by not only the organic compound, but also the chamber, and thus the transmission light intensity of the laser beam is greatly reduced. Therefore, the irradiation light intensity at which two-photon absorption occurs in the organic compound can be further easily determined.

Furthermore, according to the fine particle manufacturing method of the present invention, in the laser light irradiation step, it is preferable that a laser beam having a predetermined wavelength which is different from the light absorption band of the organic compound and acts on the solvent is irradiated to the treatment target liquid as the laser beam having a wavelength longer than the light absorption band of the organic compound.

Furthermore, in the fine particle manufacturing apparatus of the present invention, it is preferable that a laser beam having a predetermined wavelength which is different from the light absorption band of the organic compound and acts on the solvent is irradiated from the laser light source to the treatment target liquid accommodated in the chamber as the laser beam having a wavelength longer than the light absorption band of the organic compound.

According to the manufacturing method and apparatus as described above, a laser beam (preferably an infrared laser beam) having a wavelength which is different from the light absorption band of the organic compound and acts on the solvent (preferably, the wavelength absorbed by the solvent) is irradiated to the organic compound irrespective of the light absorption characteristic of the organic compound contained in the treatment target liquid to implement fine pulverization. Accordingly, the organic compound can be finely pulverized into fine particles while sufficiently preventing occurrence of photochemical reactions of the organic compound in the solvent.

In the manufacturing method and apparatus described above, when only a part of the organic component is dissolved in the solvent, that is, it is hardly-soluble or insoluble in the solvent, the organic compound can be apparently solubilized in the solvent by finely pulverizing the organic compound into fine particles with laser beam irradiation, as described above. That is, liquid containing fine particles of hardly soluble or insoluble organic compound can be manufactured.

Furthermore, in the manufacturing method and apparatus described above, it is preferable that the wavelength of the laser beam to be irradiated to the treatment target liquid is 900nm or more. Alternatively, it is preferable that the wavelength of the laser beam is set to a wavelength of the light absorption band of the solvent. Accordingly, occurrence of the photochemical reactions of the organic compound in the treatment target liquid can be reliably prevented while the fine pulverization of the organic compound is sufficiently implemented by the action of the laser beam on the solvent.

It is preferable that the irradiation light intensity of the laser beam to the treatment target liquid is set to less than the irradiation light intensity at which two-photon absorption occurs in the organic compound. When a laser beam having the irradiation light intensity at which two-photon absorption occurs in the organic compound is irradiated to the organic compound, a photochemical reaction may occur in the organic compound by the two-photon absorption regardless of use of the laser beam having a wavelength at which no photochemical reaction occurs. On the other hand, by irradiating the organic compound with a laser beam whose irradiation light intensity is less than the irradiation light intensity at which two-photon absorption occurs, occurrence of photochemical reactions in the organic compound can be reliably prevented.

Furthermore, it is preferable that the laser beam is irradiated to the treatment target liquid while cooling the treatment target liquid. Accordingly, degradation, etc., of the organic compound due to thermal decomposition when the laser beam is irradiated can be prevented.

Furthermore, it is preferable that the manufacturing method measures the absorbance of the organic compound in the treatment target liquid during irradiation of a laser beam to the treatment target liquid and monitors the fine pulverization state of the organic compound. Likewise, the manufacturing apparatus is preferably provided with monitoring light absorption band measuring means for measuring the absorbance of the organic compound in the treatment target liquid and monitors the fine pulverization state of the organic compound. In this case, since the fine pulverization state is monitored, the stop/continuation of the laser beam irradiation can be determined in accordance with the fine pulverization state, and excessive laser beam irradiation to the organic compound can be avoided.

Furthermore, in the manufacturing method described above, it is preferable that the irradiation light intensity of the laser beam irradiated to the chamber is varied while measuring the transmission light intensity of the laser beam transmitted through the treatment target liquid in the chamber, thereby determining the irradiation light intensity at which no two-photon absorption occurs in the organic compound.

When the irradiation light intensity of the laser beam irradiated to the chamber in which the treatment target liquid is accommodated is varied while measuring the transmission light intensity of the laser beam transmitted through the chamber, two-photon absorption occurs in the organic compound at the time when the irradiation light intensity reaches some irradiation light intensity. At this time, the transmission light intensity of the laser beam transmitted through the chamber is sharply varied. Therefore, the irradiation light intensity at which no two-photon absorption occurs can be easily determined. Practically, the irradiation light intensity at which no two-photon absorption occurs is used.

Furthermore, it is preferable that a stabilizer for stably dispersing in the treatment target liquid the fine particles manufactured in the treatment target liquid is added in the treatment target liquid before or during irradiation of the laser beam to the treatment target liquid. In this case, the temporarily manufactured fine particles are stably dispersed in the treatment target liquid by the stabilizer, and aggregation of the fine particles can be sufficiently prevented, so that the manufacturing efficiency of the fine particles can be enhanced. Here, it is preferable that the stabilizer is a surfactant. In this case, in addition to the enhancement of the manufacturing efficiency of the fine particles, the organic compound can be finely pulverized by irradiating the laser beam to the organic compound while more sufficiently preventing occurrence of photochemical reactions in the organic compound.

Furthermore, in the manufacturing apparatus, the laser beam source is preferably a wavelength-variable laser beam source. In this case, a laser beam having a proper wavelength can be irradiated to the treatment target liquid on the basis of the light absorption band of the organic compound, the light absorption characteristic of the solvent, etc.

Furthermore, it is preferable that the manufacturing device is further provided with a transmission light intensity measuring device for measuring the transmission light intensity of the laser beam transmitted through the treatment target liquid in the chamber, and irradiation light intensity adjusting means for adjusting the irradiation light intensity of the laser beam irradiated from the laser beam source to the chamber.

According to the construction as described above, a laser beam having a predetermined wavelength is irradiated from the laser beam source to the treatment target liquid in the chamber, and the transmission light intensity of the laser beam transmitted through the treatment target liquid is measured by the transmission light intensity measuring device. Here, when the irradiation light intensity of the laser beam is increased by the irradiation light intensity adjusting means, two-photon absorption occurs in the organic compound at some irradiation light intensity. At this time, the transmission light intensity of the laser beam is sharply varied. Accordingly, an irradiation light intensity at which no two-photon absorption occurs can be easily determined.

Here, it is preferable that the chamber more greatly absorbs a laser beam which has a wavelength longer than the light absorption band and has an irradiation light intensity at which two-photon absorption occurs in the organic compound, as compared with a laser beam having an irradiation light intensity at which no two-photon absorption occurs.

In this case, when the irradiation light intensity reaches the irradiation light intensity at which the two-photon absorption occurs in the organic compound, the laser beam is greatly absorbed by not only the organic compound, but also the chamber, and thus the transmission light intensity of the laser beam is greatly reduced. Therefore, the irradiation light intensity at which no two-photon absorption occurs in the organic compound can be more easily determined.

Furthermore, it is preferable that the organic compound contained in the treatment target liquid is a material having a relatively weak intermolecular force, for example, a material whose melting point is 250°C or less, such as medicaments. The organic compound having a low melting point as described above is easily finely pulverized by the action of the laser beam on the solvent. Accordingly, the fine pulverization of the organic compound by the laser beam irradiation can be suitably implemented.

Furthermore, when the organic compound to be finely pulverized is a medicament, the photochemical reaction of the medicament by laser beam irradiation can be sufficiently prevented. Therefore, the fine particles of the medicament can be manufactured without losing the medicinal benefits thereof. Furthermore, the surface area of the medicament is increased by the fine pulverization of the medicament, and the absorptivity thereof into the tissues of a living body is enhanced, so that the quick-acting fine particles can be obtained. Furthermore, when the medicament is hardly-soluble or insoluble in the solvent, the medicament can be apparently dissolved in the solvent. Furthermore, when the organic compound is a medicament as described above, it is preferable that water is used as the solvent. Alternatively, solvent other than water may be used.

Furthermore, a medicament in fine particle form according to the present invention is a fine particle manufactured by the above-described fine particle manufacturing method. Even in the case of hardly-soluble material or insoluble material, fine particles thereof can be apparently solubilized.

Furthermore, an injectable agent manufacturing method of the present invention is that liquid containing fine particles such as injection water containing fine particles is manufactured according to the fine particle manufacturing method described above and then the liquid concerned is added with an isotonicity agent, or injectable agent containing fine particles is manufactured under existence of an isotonicity agent according to the fine particle manufacturing method described above. According to the manufacturing method described above, a medicament which is hardly-soluble or insoluble in water can be solubilized in water while sufficiently preventing the photochemical reaction thereof. Therefore, even when the medicament is hardly-soluble or insoluble in water, it can be manufactured as an injectable agent. Furthermore, the medicament is finely pulverized into fine particles, and thus a quick-acting injectable agent for a living body can be manufactured.

Furthermore, an injectable agent according to the present invention is manufactured according to the injectable agent manufacturing method described above. In the injectable agent as described above, the medicament is finely pulverized, and the surface area thereof is increased, so that the fine particles have high absorption performance into living bodies. Therefore, the injectable agent quickly acts on a living body when it is injected into the living body.

### Brief Description of the Drawings

Fig. 1 is a diagram schematically showing a first embodiment of a fine particle manufacturing apparatus.

Fig. 2 is a flowchart showing an example of a fine particle manufacturing method.

Fig. 3 is a graph showing an absorbance characteristic of clobetasone butyrate according to Example 1.

Fig. 4 is a graph showing the absorbance characteristic of saturated solution of clobetasone butyrate before and after laser beam irradiation.

Fig. 5 is a graph showing the variation of the absorbance characteristic of clobetasone butyrate solution with respect to the irradiation time according to Example 1.

Fig. 6 is a graph showing the variation of the absorbance characteristic of clobetasone butyrate solution with respect to the time lapse after the laser beam irradiation according to Example 1.

Fig. 7 is a graph showing the absorbance characteristic of carbamazepine solution before and after laser beam irradiation according to Example 2.

Fig. 8 is a graph showing the relationship between the addition concentration of a surfactant and the absorbance characteristic of carbamazepine solution according to Example 3.

Fig. 9 is a graph showing the relationship between the addition concentration of a surfactant and the absorbance characteristic of clobetasone butyrate solution according to Example 4.

Fig. 10 is a block diagram schematically showing the construction of a second embodiment of the fine particle manufacturing apparatus.

Fig. 11 is a flowchart showing another example of the fine particle manufacturing method.

Fig. 12 is a table showing typical absorption peak wavelengths and absorbance of solvent.

Fig. 13 is a graph showing the wavelength dependence of the absorbance of ethyl alcohol.

Fig. 14 is a graph showing the wavelength dependence of the absorbance of polyethylene glycol 400.

Fig. 15 is a graph showing the wavelength dependence of the absorbance of glycerol.

Fig. 16 is a graph showing the wavelength dependence of the absorbance of clobetasone butyrate suspended solution before and after a fine pulverization treatment.

Fig. 17 is a graph showing the laser beam wavelength dependence of clobetasone butyrate purity after the fine pulverization treatment.

Fig. 18 is a graph showing the absorption characteristic of clobetasone butyrate in an infrared wavelength region.

Fig. 19 is a graph showing the laser beam wavelength dependence of the fine pulverization efficiency.

Fig. 20 is a block diagram schematically showing the construction of a modified example of the fine particle manufacturing apparatus.

Fig. 21 is a flowchart showing another example of the fine particle manufacturing method.

### Best Modes for Carrying Out the Invention

Preferable embodiments of fine particles, method and apparatus for manufacturing the fine particles, injectable agent and a method for manufacturing the injectable agent will be described below in detail with reference to the drawings. In the description of the drawings, the same elements are represented by the same reference symbols, and overlapping description thereof is omitted. The ratio in dimension of the drawings are not necessarily coincident with that in the description.

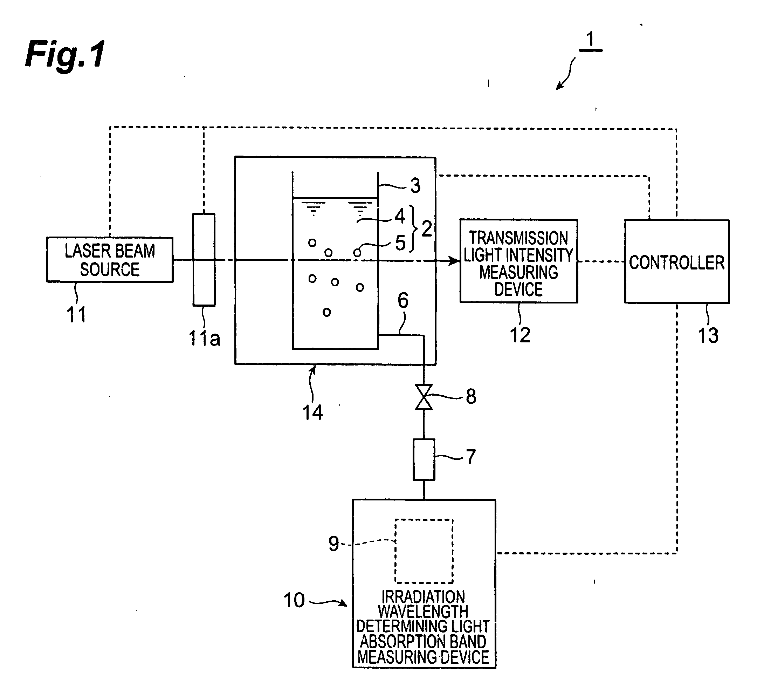

Fig. 1 is a schematic diagram showing a first embodiment of a fine particle manufacturing apparatus according to the present invention. As shown in Fig. 1, a fine particle manufacturing apparatus 1 has a chamber 3 for accommodating treatment target liquid 2 therein. The chamber 3 is formed of quartz or the like. The treatment target liquid 2 comprises water 4 and a hardly-soluble medicament 5 suspended in water 4, and the hardly-soluble medicament 5 comprises dissolved material which is very slightly dissolved in water 4, and a non-dissolved material (solid material) which is not dissolved in water 4.

It is preferable that the hardly-soluble medicament 5 is a hardly-soluble medicament which is hardly soluble in water 4 and in which at least a part of the light absorption band (ultraviolet light absorption band) is longer in wavelength than the ultraviolet light absorption band of water itself. As the hardly-soluble medicament 5, clobetasone butyrate which is an adrenal cortex hormone, carbamazepine, ibuprofen, for example, may be mentioned.

A drain pipe 6 for draining the treatment target liquid 2 from the chamber 3 is connected to the lower side of the chamber 3. In the drain pipe 6 are provided a valve 8, and a separation filter 7 for transmitting therethrough the treatment target liquid 2 discharged from the chamber 3 and separating the non-soluble material of the hardly-soluble medicament 5 from the treatment target liquid 2. The fine particle manufacturing apparatus 1 is provided with an irradiation wavelength determining light absorption band measuring device 10 containing a light absorption band analyzing chamber 9. The drain pipe 6 is connected to the light absorption band analyzing chamber 9 of the irradiation wavelength determining light absorption band measuring device 10. Accordingly, when the valve 8 is opened, a part of the treatment target liquid 2 in the fine particle manufacturing chamber 3 is drained from the chamber 3 through the drain pipe 6, the non-dissolved material of the hardly-soluble medicament 5 is separated from the treatment target liquid 2 by the separation filter 7, the treatment target liquid 2 containing the dissolved material transmitted through the separation filter 7 is introduced to the light absorption band analyzing chamber 9, and the light absorption band of the dissolved material dissolved in the water 4 is measured by the irradiation wavelength determining light absorption band measuring device 10.

As described above, the manufacturing apparatus 1 is provided with the irradiation wavelength determining light absorption band measuring device 10. Therefore, even in the case of hardly-soluble medicament 5 whose light absorption band is unknown, the treatment target liquid 2 discharged from the chamber 3 is introduced into the light absorption band analyzing chamber 9, and the light absorption band can be immediately measured. The non-dissolved material can be reliably removed from the treatment target liquid 2 introduced into the light absorption band analyzing chamber 9 by the separation filter 7, and thus the light absorption band of the dissolved material can be reliably measured. The irradiation wavelength determining light absorption band measuring means is constructed by the drain pipe 6, the separation filter 7, the valve 8 and the light absorption band measuring device 10.

Furthermore, the fine particle manufacturing device 1 is provided with a wavelength-variable laser 11 which can vary the wavelength of a laser beam while irradiating the laser beam to the hardly-soluble medicament 5 in the chamber 3, and irradiation light intensity adjusting means 11a for adjusting the irradiation light intensity of the laser beam emitted from the wavelength-variable laser 11. The wavelength-variable laser 11 can emit a laser beam having a wavelength longer than the light absorption band of the hardly-soluble medicament 5. An attenuating filter having a high optical pressure resistance or an optical attenuator using optical interference/reflection may be used for example as the irradiation light intensity adjusting means 11a. Furthermore, a transmission light intensity measuring device 12 for measuring the transmission light intensity of a laser beam which is emitted from the wavelength-variable laser 11 and transmitted through the chamber 3 is disposed at the opposite side to the wavelength-variable laser 11 with respect to the chamber 3.

The fine particle manufacturing device 1 is provided with a monitoring light absorption band measuring device 14 which can measure the light absorption band in the chamber 3. The monitoring light absorption band measuring device 14 is provided with a box for accommodating the chamber 3 therein, and a spectroscopic light source and a photodetector which are provided in the box, and it can measure the absorbance of the organic compound of the treatment target liquid 2 in the chamber 3 and monitor the fine pulverization state of the hardly-soluble medicament. Furthermore, a laser beam passage port is formed in the box so that a laser beam emitted from the wavelength-variable laser 11 is passed through the chamber 3 and reaches the transmission light intensity measuring device 12. The monitoring of the light absorption band variation of the treatment target liquid 2 by the monitoring light absorption band measuring device 14 as described above is important to determine an excellent laser beam irradiation time to the treatment target liquid 2, and it serves to avoid excessive laser beam irradiation to the hardly-soluble medicament 5.

Furthermore, a controller 13 is electrically connected to the irradiation wavelength determining light absorption band measuring device 10, the wavelength-variable laser 11, the monitoring light absorption band measuring device 14, the irradiation light intensity adjusting means 11a and the transmission light intensity measuring device 12. The controller 13 controls the irradiation wavelength determining light absorption band measuring device 10, the wavelength-variable laser 11, the monitoring light absorption band measuring device 14, the irradiation light intensity adjusting means 11a and the transmission light intensity measuring device 12.

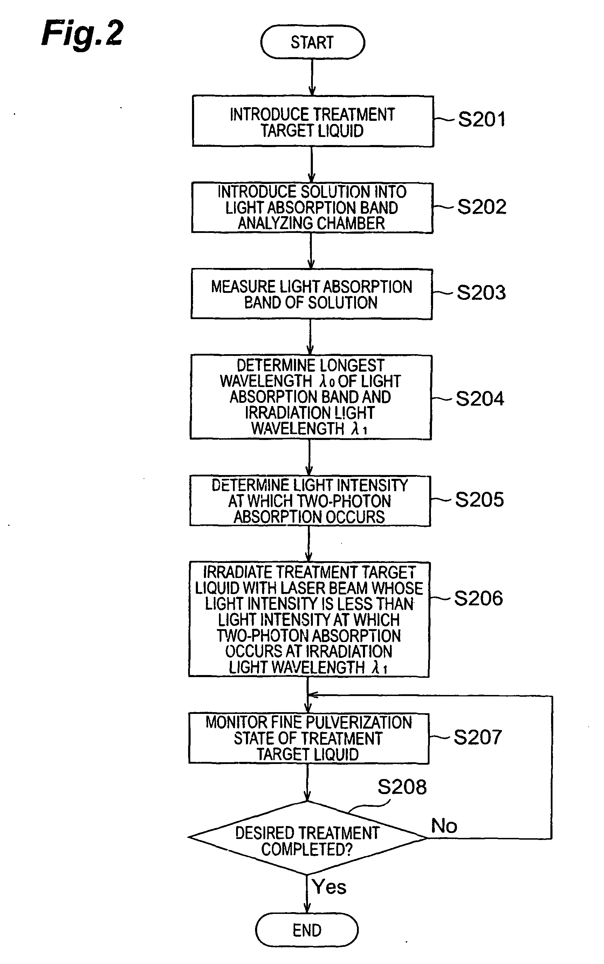

Next, a method for manufacturing fine particles by using the fine particle manufacturing apparatus 1 will be described with reference to the flowchart of Fig. 2.

First, the water 4 and the hardly-soluble medicament 5 are mixed with each other, and then stirred to prepare the treatment target liquid 2 (preparation step). In the treatment target liquid 2, a part of the hardly-soluble medicament 5 is dissolved in the water 4 by stirring to be dissolved material, and the residual is not dissolved in the water 4 to be non-dissolved material.

Subsequently, the treatment target liquid 2 is introduced into the fine particle manufacturing chamber 3 (S201). At this time, the valve 8 disposed in the drain pipe 6 is opened by the controller 13, and a part of the treatment target liquid 2 is drained from the chamber 3 to the drain pipe 6. In the separation filter 7, the non-dissolved material of the hardly-soluble medicament 5 is separated from the treatment target liquid 2, and the residual is introduced as the dissolved liquid into the light absorption band analyzing chamber 9 (S202).

Subsequently, the light absorption band of the dissolved material of the hardly-soluble medicament 5 in the dissolved liquid introduced in the light absorption band analyzing chamber 9 is measured by the light absorption band measuring device 10. A measurement result of the light absorption band is transmitted to the controller 13, and the longest wavelength λ₀ is determined on the basis of the measurement result of the light absorption band for the dissolved material in the controller 13 (S203). Here, the longest wavelength λ₀ of the light absorption band is defined as a wavelength which is located at the base of the mountain at the long wavelength side of the light absorption band in the absorbance characteristic, and at which variation of the absorbance which clearly seems to be electron transition absorption of the dissolved material can be observed, as compared with the absorbance of the visible light region in the longer wavelength region.

After the longest wavelength λ₀ is determined as described above, a wavelength longer than the longest wavelength λ₀ is determined as a laser beam irradiation wavelength λ₁ used for the fine particle manufacture described later. Then, the wavelength-variable laser 11 is controlled by the controller 13, and the irradiation wavelength of the laser beam is set to the laser beam irradiation wavelength λ₁ thus determined in the wavelength-variable laser 11 (S204). At this time, when the hardly-soluble medicament 5 is clobetasone butyrate, the laser beam irradiation wavelength λ₁ is preferably longer than the longest wavelength λ₀ by 70nm or more. In this case, the photochemical reaction in the hardly-soluble medicament 5 can be sufficiently prevented.

Subsequently, the irradiation light intensity of the laser beam in the fine particle manufacturing process is determined keeping the laser beam irradiation wavelength λ₁. First, a laser beam is irradiated to the fine particle manufacturing chamber 3 by the wavelength-variable laser 11, and the transmission light intensity of the laser beam transmitted through the fine particle manufacturing chamber 3 is measured by the transmission light intensity measuring device 12. The irradiation light intensity of the laser beam irradiated to the chamber 3 is varied by the irradiation light intensity adjusting means 11 a while the transmission light intensity of the laser beam transmitted through the fine particle manufacturing chamber 3 is measured by the transmission light intensity measuring device 12. The relationship between the irradiation light intensity of the laser beam and the transmission light intensity of the laser beam is obtained. Here, when two-photon absorption occurs in the hardly-soluble medicament 5, the sharp reduction of the transmission light intensity of the laser beam is observed. Accordingly, the irradiation light intensity at which the two-photon absorption occurs in the hardly-soluble medicament 5 can be easily determined. The irradiation light intensity adjusting means 11a is controlled by the controller 13, and the irradiation light intensity of the laser beam is adjusted by the transmission light intensity adjusting device 12 so as to be equal to an irradiation light intensity smaller than the irradiation light intensity thus determined at which two-photon absorption occurs (S205).

Under this state, the wavelength-variable laser 11 is actuated by the controller 13 to irradiate the laser beam from the wavelength-variable laser 11 to the fine particle manufacturing chamber 3, whereby the hardly-soluble medicament 5 is finely pulverized, and fine particles of the hardly-soluble medicament 5 are manufactured (S206, the laser beam irradiation step).

Here, when the hardly-soluble medicament 5 is a medicinal drug, the treatment is required to be carried out so as to avoid excessive laser beam irradiation in the manufacturing process of fine particles. Therefore, with respect to the treatment target liquid 2, the variation of the absorbance of the treatment target liquid 2 with respect to the laser beam irradiation time is measured by the monitoring light absorption band measuring device 14 (S207), and it is judged whether the desired treatment is completed (S208). If the desired treatment is completed, the laser beam irradiation is stopped. If the desired treatment is not completed, the laser beam irradiation is continued. Specifically, the completion of the desired treatment is judged by irradiating a laser beam to the treatment target liquid 2 from the wavelength-variable laser 11 and measuring the absorbance variation measured by the monitoring light absorption band measuring device 14. If the time variation of the light absorption band is hardly observed, the completion of the desired treatment is judged. The treatment time may be set to the time period from the start time of the laser beam irradiation untill the time at which the light absorption band hardly varies with respect to the laser beam irradiation time.

By finely pulverizing the hardly-soluble medicament 5 as described above, the hardly-soluble medicament 5 can be apparently dissolved in the water 4. Even when the hardly-soluble medicament 5 is finely pulverized, the solubility state of the hardly-soluble medicament 5 in the water 4 can be kept stable for a long time. Furthermore, a laser beam having a wavelength longer than the longest wavelength in the light absorption band of the hardly-soluble medicament 5 is used as the laser beam. Therefore, even when the laser beam is irradiated to the hardly-soluble medicament 5, the photochemical reaction thereof can be sufficiently prevented, and the hardly-soluble medicament 5 can be sufficiently prevented from being degenerated. Accordingly, the fine particles thereof can be obtained without losing the medicinal benefits of the hardly-soluble medicament 5.

Furthermore, the laser beam whose irradiation light intensity is less than the irradiation light intensity at which two-photon absorption occurs is irradiated to the hardly-soluble medicament 5, whereby the photochemical reaction occurring in the hardly-soluble medicament 5 can be sufficiently prevented, and the degeneration of the hardly-soluble medicament 5 can be more sufficiently prevented.

The fine particles of the hardly-soluble medicament 5 thus obtained can be apparently solubilized in water, and also sufficiently keeps the medicinal benefits thereof. Therefore, there can be performed physical and chemical researches and searching and determination of candidate compounds such as screening, etc., the ADME test (absorption, distribution, metabolism, excretory test), general drug toxicity, general pharmacology, pharmacology, biochemical research in preclinical tests of animals, clinical tests, etc., which have not been able to be estimated in the conventional manner before the fine pulverization of the hardly-soluble medicament 5 is completed. Accordingly, even when an achieved compound library or newly synthesized medicaments or natural products are hardly soluble in water, the capital investment thereof is not wasted. Furthermore, the fine particles of the hardly-soluble medicament 5 have a large surface area which is sufficiently larger as compared with the state before it is finely pulverized. Accordingly, absorbability into the tissues of living bodies is enhanced, and it can quickly act on living bodies.

Furthermore, according to the fine particle manufacturing method described above, medicaments which can be administered to remarkably various kinds of living bodies can be achieved, and thus the selectivity of medicaments to be administered can be remarkably enlarged.

In the fine particle manufacturing method described above, it is preferable that stabilizer for stably dispersing the fine particles of the medicament is added to the treatment target liquid 2 before or during the irradiation of the laser beam. By adding the stabilizer to the treatment target liquid 2 as described above, the hardly-soluble medicament 5 is stably dispersed in the water 4 by the stabilizer, and thus the manufacturing efficiency of fine particles can be enhanced. The stabilizer is preferably a surfactant. In this case, in addition to the enhancement of the manufacturing efficiency of the fine particles, the hardly-soluble medicament 5 can be finely pulverized with preventing the photochemical reaction of the hardly-soluble medicament 5 more sufficiently even when a laser beam having a wavelength longer than the irradiation wavelength is irradiated to the hardly-soluble medicament 5.

Any material may be used as the stabilizer insofar as it has a property of dispersing the hardly-soluble medicament 5 in water 4 and also has no adverse effect on the living body, and as such materials described in the "MEDICINAL ADDITIVE DICTIONARY" or "MEDICINAL ADDITIVE HANDBOOK," may be used, for example, Tween20, Tween60, Tween80, Tween85, sorbitan trioleate, sorbitan monolaurate, sorbitan monopalmitate, sorbitan monostearate, polyoxyethylene, sorbitan monopalmitate, triethanol amine, cyclodextrin, albumin, etc.

As described above, it is effective to use a surfactant in the fine pulverization process of medicaments, however, it is not desirable that the surfactant exists after the fine pulverization of the medicament. Therefore, it is preferable that, for example, the treatment target liquid 2 is diluted to separate the fine particles from the surfactant, and aggregating fine particles as aggregate of the fine particles can be achieved. Here, the aggregating fine particles can be achieved by a separating method such as centrifugal separation or the like. The aggregating fine particles achieved after the fine particles are manufactured can be easily treated in a re-dispersing process.

In the above-described manufacturing method, the absorbance variation of the treatment target liquid 2 is measured in the fine particle manufacturing process by the monitoring light absorption band measuring device 14, and the laser beam irradiation is stopped when the desired treatment is completed. However, the treatment time of the laser beam irradiation to the same treatment target liquid as the treatment target liquid 2 may be predetermined before the fine particles are manufactured. The determination of the treatment time may be carried out by measuring the light absorption band of the organic compound by the monitoring light absorption band measuring device 14 and setting the treatment time to the time period from the start time of the laser beam irradiation to the time at which the time-variation of the light absorption band is hardly observed. However, when the treatment time is predetermined before the fine particles are manufactured, the laser beam irradiation may be stopped in the fine particle manufacturing process at the time when the treatment time has elapsed, and it is not necessary to monitor the fine pulverization state of the medicament in the treatment target liquid 2 by the monitoring light absorption band measuring device 14 during the fine particle manufacturing process.

Next, an embodiment of the method for manufacturing the injectable agent according to the present invention will be described.

First, liquid containing fine particles of hardly-soluble medicament 5 which is apparently solubilized in injection water 4 is manufactured by using the fine particle manufacturing apparatus 1 described above. This liquid manufacturing method is similar to the fine particle manufacturing method as described above. Stabilizer may be added to the treatment target liquid 2 before or during laser beam irradiation of the hardly-soluble medicament 5 as in the case of the fine particle manufacturing method.

Subsequently, an isotonicity agent is added to this liquid to manufacture the injectable agent. Here, the isotonicity agent has a function of adjusting the osmotic pressure of the injectable liquid so that the injectable liquid is equal to the blood of a living body in osmotic pressure, and, for example, sucrose, physiological salt solution or the like may be used as the isotonicity agent.

According to this manufacturing method, the hardly-soluble medicament 5 can be solubilized in the injection water 4 while sufficiently preventing the photochemical reaction thereof. Therefore, even the hardly-soluble medicament 5 can be manufactured as an injectable agent. Furthermore, since the hardly-soluble medicament 5 is finely pulverized into fine particles, an injectable agent quickly acting on living bodies can be manufactured.

The injectable agent thus manufactured contains fine particles of medicament which sufficiently keeps the medicinal benefits of the hardly-soluble medicament 5, and thus the injectable agent exhibits the same medicinal benefits as the hardly-soluble medicament 5 insofar as the hardly-soluble medicament 5 itself is harmless to living bodies. Furthermore, the hardly-soluble medicament 5 is finely pulverized and thus the surface area of the fine particles is increased, so that the fine particles have high absorbility to living bodies. Therefore, the injectable agent works quickly when it is injected into a living body.

In the manufacturing apparatus 1 described above, the controller 13 controls the irradiation wavelength determining light absorption band measuring device 10, the wavelength-variable laser 11, the monitoring light absorption band measuring device 14, the irradiation light intensity adjusting means 11a and the transmission light intensity measuring device 12, however, the control device 13 is not necessarily required. Accordingly, an operator may control the irradiation wavelength determining light-absorption band measuring device 10, the wavelength-variable laser 11, the monitoring light absorption band measuring device 14, the irradiation light intensity adjusting means 11a and the transmission light intensity measuring device 12.

In the above manufacturing apparatus 1, the material of the fine particle manufacturing chamber 3 is quartz. However, it is not limited to quartz, and any material may be used for the chamber 3 insofar as it more greatly absorbs the laser beam having the irradiation light intensity at which two-photon absorption occurs in the hardly-soluble medicament 5 as compared with the laser beam having the irradiation light intensity at which no two-photon absorption occurs. In addition to quartz, for example, synthesized quartz, ultraviolet-ray transmissible glass, ultraviolet-ray transmissible polymer (polymer), etc., may be used as the material of the chamber 3.

Furthermore, in this embodiment, water is used as the solvent in the treatment target liquid 2 to measure the light absorption band of the hardly-soluble medicament 5 by the irradiation wavelength determining light absorption band measuring device 10, however, the present invention is not limited to water. For example, water-soluble organic solvent such as ethanol, propylene glycol, polyethylene glycol or the like, or vegetable oil may be used.

Furthermore, when some medicament is an insoluble medicament which is never dissolved in water, that is, whose light absorption band cannot be measured in water, in order to enable dissolution of a part of the medicament and the measurement of the light band absorption thereof, organic solvent such as ethylene alcohol, acetone, dimethyl sulfoxide or the like, or a mixture liquid of the organic solvent and water is used in place of water, and the light absorption band of the above material is separately measured by a spectrophotometer to determine a proper laser beam irradiation wavelength for the manufacture of fine particles.

When organic solvent is used, there is a tendency that the longest wavelength of the light absorption band is shifted as compared with the case where water is used. Therefore, when the light absorption band of a medicament is measured, it is preferable that a mixture liquid of organic solvent and water is used. Furthermore, when a laser beam is irradiated to a medicament to manufacture the fine particles of the medicament, water is required to be used as solvent from the point of view of preventing an adverse effect on living bodies.

Furthermore, in the above embodiment, a hardly-soluble or insoluble medicament such as clobetasone butyrate, carbamazepine or the like is used as the medicament, however, the medicament of the present invention is not limited to these hardly-soluble or insoluble medicaments.

Furthermore, in the above embodiment, clobetasone butyrate and carbamazepine which are medicinal drugs are used as the medicaments, however, the fine particle manufacturing method and the injection liquid manufacturing method for the present invention is applicable to not only the above medicinal drugs, but also medicinal drug candidates (natural products, compound library, etc.), quasi-drugs, cosmetic preparations, etc.

In the above embodiment, the light absorption band of the hardly-soluble medicament 5 is measured when the fine particles of the hardly-soluble medicament 5 are manufactured. However, when the light absorption band of the hardly-soluble medicament 5 is known in advance, it is not necessary to measure the light absorption band of the hardly-soluble medicament 5. Therefore, the light absorption band measuring device 10 and the transmission light intensity measuring device are not needed. However, the controller 13 is needed to determine the proper irradiation time on the basis of the monitoring light absorption band measurement, and thus the monitoring light absorption band measuring device 14 is needed. Here, the wavelength-variable laser 11 may be directly used in the laser beam irradiation time, however, a wavelength-fixed laser for emitting a laser beam having a wavelength longer than the light absorption band of the hardly-soluble medicament 5 may be used in place of the wavelength-variable laser 11.

Next, the content of the present invention will be described more specifically on the basis of examples, however, the present invention is not limited to the examples.

(Example 1)

Fine pulverization of clobetasone butyrate which is adrenal cortex hormone as hardly-soluble medicament was attempted.

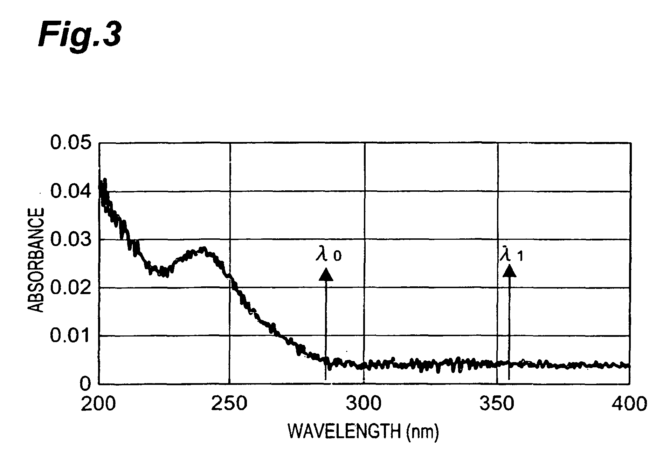

First, powder of clobetasone butyrate was suspended in water and left for ten minutes. Thereafter, it was passed through a filter having a mesh of 1 µm to obtain solution in which a minute amount of clobetasone butyrate is dissolved (clobetasone butyrate solution). The absorbance characteristic of the solution was measured by using a general-purpose spectrophotometer (HITACHI U-3500). Fig. 3 shows the absorbance characteristic of the solution. The optical path length was set to 10mm for the measurement. From the absorbance characteristic shown in Fig. 3, it was apparent that the longest wavelength λ₀ of the light absorption band was in the vicinity of 280nm.

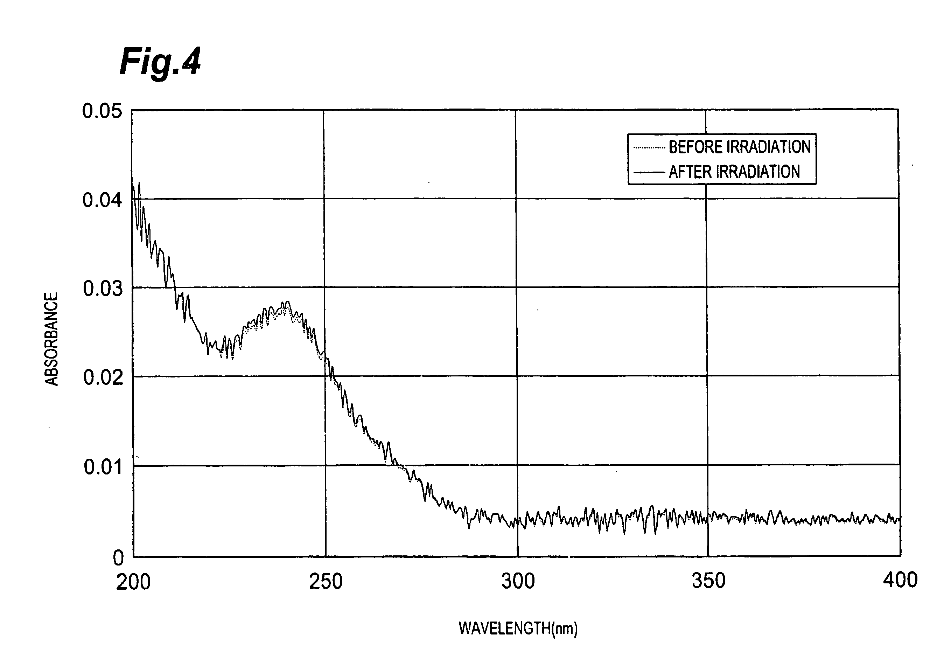

Subsequently, a laser beam having an irradiation light intensity at which no two-photon absorption occurs (λ₁=355nm, 380mJ/cm²Pulse, FWHM=4ns, 20Hz, irradiation time: 15 minutes) was continuously irradiated the treatment target liquid containing the powder of clobetasone butyrate. At this time, the laser beam irradiation wavelength was set to 355nm for the reason that it was expected as shown in Fig. 4, no variation of the absorbance characteristic of the saturated water solution of clobetasone butyrate was observed before and after light irradiation of 355nm (YAG triple higher harmonics), and thus if a wavelength longer than the longest wavelength λ₀ by 70nm is selected, photochemical reactions could be avoided.

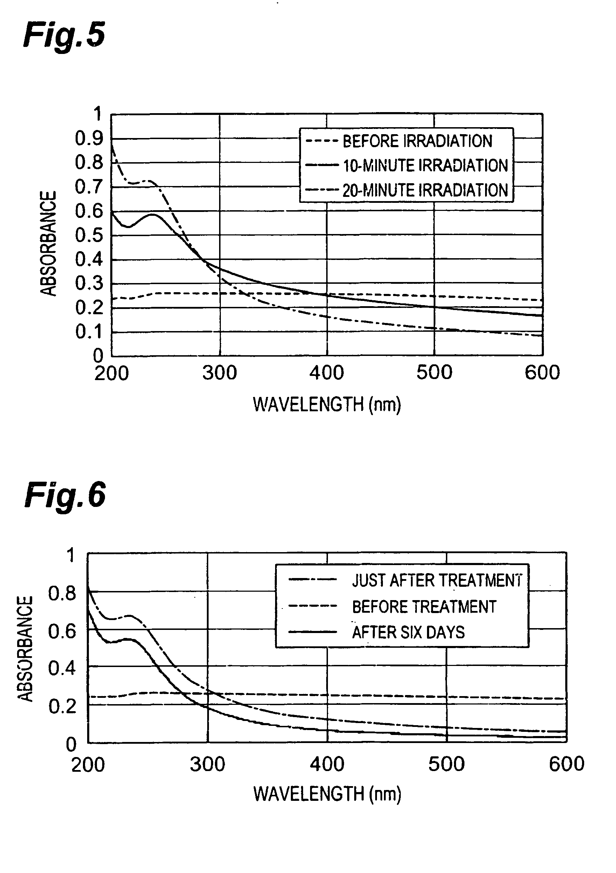

Fig. 5 shows a measurement result of the absorbance characteristic before and after the laser beam irradiation. As indicated by the dashed line of Fig. 5, only the scattering loss caused by the suspension of the clobetasone butyrate powder (the characteristic having no wavelength dependence) was observed before the irradiation. However, as indicated by the solid line and dot-dashed line of Fig. 5, the absorbance characteristic of clobetasone butyrate itself appears as the irradiation time is increased. This is a state where the inherent property of the material is observed in the solution, and the particles of clobetasone butyrate are finely pulverized. In Fig. 5, the solid line represents the absorbance characteristic measured in 10 minutes after the laser beam irradiation, and the dot-dashed line represents the absorbance characteristic measured in 20 minutes after the laser beam irradiation.

Furthermore, observing the solution after the laser beam irradiation, the solution was transparent. It was apparent from this observation that clobetasone butyrate serving as a hardly-soluble medicament was apparently dissolved.

When the variation of the absorbance characteristic after the laser beam irradiation with respect to the time lapse was measured with respect to the finely-pulverized solution, it was observed that precipitation caused by the aggregation of fine particles was rarely observed after six days as shown in Fig. 6 and the stability was relatively high. Furthermore, the ultraviolet ray absorption curves just after the treatment (dot-dashed line) and after six days (solid line) shown in Fig. 6 were similar to each other, and they were similar to the characteristic of dissolved clobetasone butyrate of Fig. 3. Therefore, it can be judged that no degeneration occurred in clobetasone butyrate after the treatment. That is, no variation is observed in the absorbance characteristic before and after irradiation at 355nm (YAG triple higher harmonics), and if a wavelength longer than the longest wavelength λ₀ by about 70nm is selected, photochemical reactions could be avoided even in the case of light irradiation having high intensity. The absorbance characteristic indicated by the dashed line is the one before laser beam irradiation.

As described above, it is apparent that the longest wavelength λ₀ is determined from the light absorption band measurement of a solution of minute amount of clobetasone butyrate, a wavelength of 355nm longer than the longest wavelength λ₀ is selected for fine pulverization, and the fine pulverization treatment is implemented at an irradiation light intensity at which no two-photon absorption occurs. Furthermore, it is also apparent that fine particles remained stable for a relatively long time under a dispersed state in solution.

(Comparative Example 1)

A laser beam was irradiated to clobetasone butyrate solution in the same manner as example 1 except that a Kr-F laser for emitting a laser beam of 248nm is used as the laser beam source. As a result, a variation in the absorbance characteristic before and after the laser beam irradiation was observed. That is, it was known that some photochemical reaction occurs at that wavelength.

(Example 2)

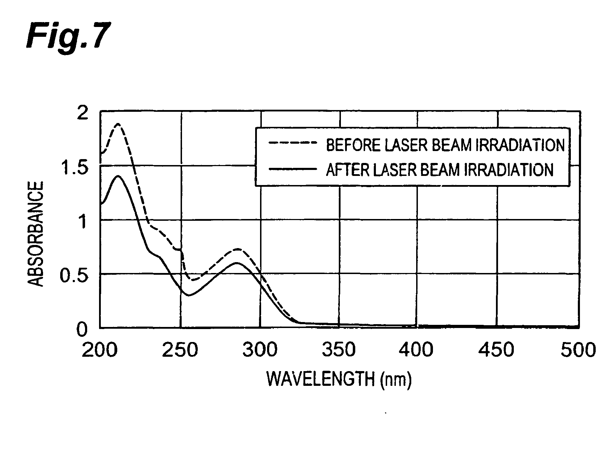

Carbamazepine powder was dispersed in water, and particles floated in water were removed by centrifugal separation after being sufficiently stirred, thereby preparing saturated solution of carbamazepine. The ultraviolet absorbance characteristic of the saturated solution was measured in the same manner as example 1 except that the optical path lengh was set to I mm. The measurement result is indicated by the dashed line of Fig. 7. As shown in Fig. 7, it is apparent that there is hardly any absorption at wavelengths of 320nm or more in the ultraviolet absorbance characteristic of carbamazepine.

Subsequently, carbamazepine was suspended in water so that the concentration thereof was equal to 2mg/ml, thereby preparing suspended liquid, and triple higher harmonics of YAG laser (λ₁=355nm, 430mJ/cm²Pulse, FWHM=4ns, 20Hz, irradiation time: 15 minutes) was irradiated to the suspended liquid. As a result, the treatment target liquid fell into a cloudy state, so that a precipitate having a very large volume appeared. This precipitate was generated by aggregation and precipitation of fine particles while containing a large amount of water molecules. When a part of the precipitate was taken out and suspended in pure water, it was instantaneously dissolved although a sample before the treatment was hardly dissolved. This is estimated as a phenomenon that the sample was pulverized by the laser beam and the particle diameter was reduced to thereby enhance the solubility. Therefore, it was considered that carbamazepine was pulverized by the laser beam irradiation and the particle diameter was reduced.

Subsequently, the precipitate after the treatment was dissolved in water until it was nearly saturated, and the ultraviolet ray absorption characteristic of the solution was measured. The result is indicated by solid line of Fig. 7. As shown in Fig. 7, as compared with the ultraviolet ray absorption characteristics of the solution before and after the laser beam irradiation, the ultraviolet ray absorption characteristics of both the cases were very similar to each other, and little photochemical reaction occurred to the extent that it did not induce any problem.

From the foregoing matter, it is apparent that the optical pulverization of carbamazepine can be performed without any photochemical reaction under the laser beam irradiation condition described above.

(Example 3)

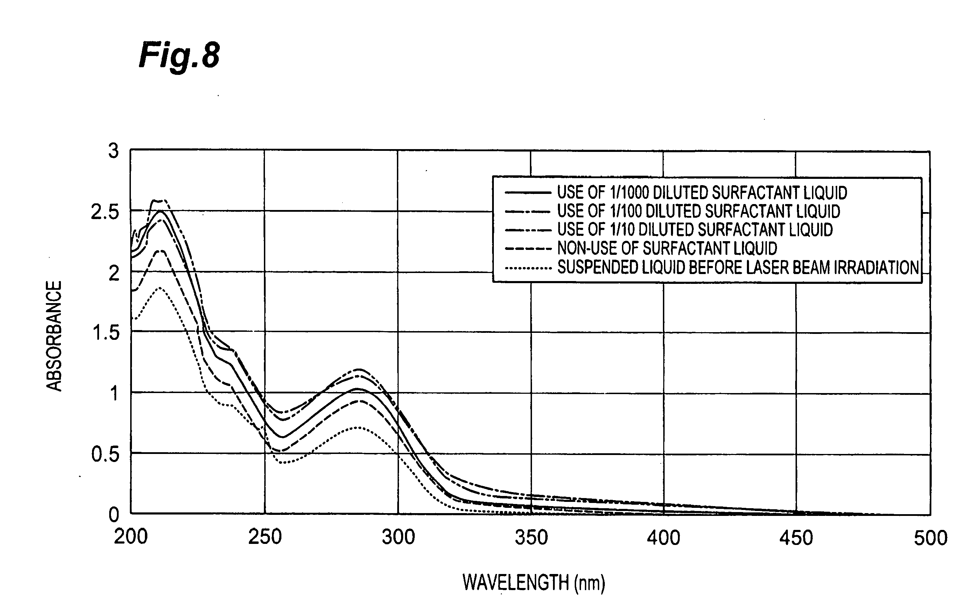

Carbamazepine was suspended in water to prepare 2ml of suspended liquid having a concentration of 1 mg/ml, and the suspended liquid was put in a quartz square cell (1cm x 1cm) and irradiated with a laser beam for fine pulverization. Laser beam irradiation was carried out with triple higher harmonics of YAG laser (λ₁=355nm, 310mJ/cm²Pulse, FWHM-4ns, 20Hz) for 15 minutes. After the laser beam irradiation, the absorbance (A1) of carbamazepine was measured by the general-purpose spectrophotometer used in example 1. The result is indicated by the dashed line of Fig. 8.

Subsequently, an effect on fine pulverization when a surfactant was added was investigated. Tween20 was used as the surfactant, and it was diluted with water so that concentrations of 1/1000, 1/100 and 1/10 of undiluted solution were prepared, thereby preparing a surfactant liquid. The above suspended liquid of 1.9ml and the surfactant liquid having each concentration were mixed, and each treatment target liquid of 2ml was prepared. In the same manner as described above, a laser beam was irradiated to each treatment target liquid to determine the relationship between the concentration of the surfactant after the laser beam irradiation and the estimated absorbance (A1). The result is shown in Fig. 8. In Fig. 8, the solid line represents the absorbance characteristic of the treatment target liquid using the surfactant liquid having the concentration of 1/1000, the one-dot-dashed line represents the absorbance characteristic of the treatment target liquid using the surfactant liquid having the concentration of 1/100, and the two-dot-dashed line represents the absorbance characteristic of the treatment target liquid using the surfactant liquid having the concentration of 1/10. The dotted line represents the absorbance characteristic of the suspended liquid before the laser beam irradiation.

The measured value (R) of the absorbance measured by the general-purpose spectrophotometer contains the light absorption (A1) of finely-pulverized carbamazepine itself, the light scattering (S) and the light absorption (A2) of the added surfactant. Al represents the fine pulverization state of carbamazepine, and it is estimated that the average particle diameter of carbamazepine is smaller as the light absorption is larger. Therefore, in order to estimate the particle diameter of carbamazepine after the treatment, with respect to the absorbance on the ordinate axis in Fig. 8, the increment (S) of the absorbance by the light scattering at each wavelength is approximated to the measured value (S1) of 500nm at which carbamazepine has no absorption, and the measured value R of the absorbance is corrected to the absorbance A1 by using the operation of A1≈R-A2-S1.

There is a tendency that as the addition concentration of the surfactant is higher, the absorbance of carbamazepine itself appears more greatly as shown in Fig. 8, and thus it is estimated that the addition of the surfactant has an effect of enhancing the fine pulverization efficiency of carbamazepine. Furthermore, since the absorbance of carbamazepine which is subjected to the fine pulverization treatment is larger than the absorbance characteristic of the saturated solution of carbamazepine, it is estimated that the particle diameter of carbamazepine is reduced to a sub-micron or less by the fine pulverization treatment. Furthermore, in both the cases where no surfactant is added and where surfactant is added, the shape of the absorbance characteristic curve is similar, so that it is considered that no photochemical reaction is induced by the laser beam irradiation.

(Example 4)

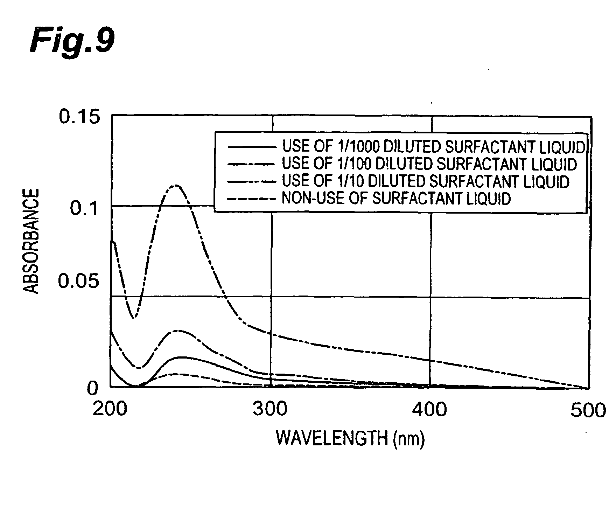

A laser beam was irradiated to treatment target liquid in the same manner as the example 3 except that clobetasone butyrate was used in place of carbamazepine. The absorbance characteristic after the laser beam irradiation was measured in the same manner as example 3. The result is shown in Fig. 9. In Fig. 9, the solid line represents the absorbance characteristic of the treatment target liquid using the surfactant liquid having the concentration of 1/1000, the one-dot-dashed line represents the absorbance characteristic of the treatment target liquid using the surfactant liquid having the concentration of 1/100, and the two-dot-dashed line represents the absorbance characteristic of the treatment target liquid using the surfactant liquid having the concentration of 1/10. The dashed line represents the absorbance characteristic of the treatment target liquid using no surfactant.

As shown in Fig. 9, with this treatment target liquid, the laser beam irradiation intensity of about 400mJ/cm²Pulse is needed to finely pulverize clobetasone butyrate, however, the actual laser beam irradiation intensity is equal to 310mJ/cm²Pulse, and thus the light absorption of clobetasone butyrate itself, that is, the fine pulverization is not observed when no surfactant is added. However, there is a tendency that as the added concentration of the surfactant is increased, the light absorption appears more greatly, and thus it is estimated that fine particles having a particle diameter of sub-micron or less are generated.

As described above, the addition of the surfactant has an effect of enhancing the efficiency of the fine pulverization treatment for clobetasone butyrate as in the case of the carbamazepine of example 3. However, it is also considered that the addition of the surfactant further has an effect of reducing the threshold value of the light irradiation intensity at which the fine pulverization phenomenon occurs. The reduction of the threshold value of the fine pulverization phenomenon is particularly effective to avoid photochemical reactions.

The fine particle manufacturing method and apparatus according to the present invention will be further described.

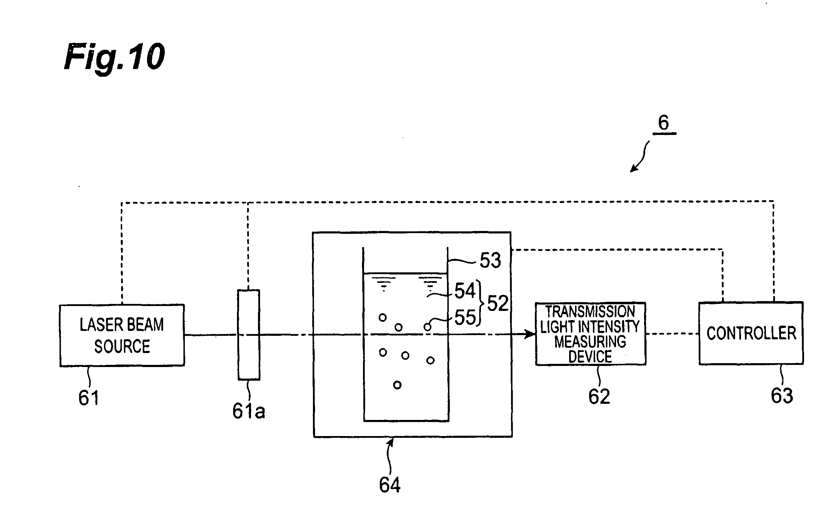

Fig. 10 is a block diagram schematically showing the construction of a second embodiment of the fine particle manufacturing apparatus according to the present invention. As shown in Fig. 10, the fine particle manufacturing apparatus 6 has a chamber 53 for accommodating treatment target liquid 52. The chamber 53 is made of quartz, for example. The treatment target liquid 52 comprises water 54 as solvent, and a hardly-soluble medicament 55 which is an organic compound suspended in water 54. The hardly-soluble medicament 55 comprises dissolved material which is extremely slightly dissolved in water 54, and non-dissolved material (solid material) which is not dissolved by water 54. As the hardly-soluble medicament 55, clobetasone butyrate as a steroid external medicine, carbamazepine as an anti-epileptic medicine, ibuprofen as an analgesic agent, etc., for example, may be mentioned.

The fine particle manufacturing apparatus 6 has a laser beam source 61 for irradiating a laser beam having a predetermined wavelength to the treatment target liquid 52 in the chamber 53. The laser beam source 61 is a light source for emitting a laser beam which has a wavelength different from the light absorption band of a medicament 55 serving as an organic compound to be finely pulverized (preferably, a wavelength longer than the light absorption band) and also acts on water 54 as solvent (preferably, a wavelength which water 54 absorbs). When the wavelength to be set to the laser beam is known in advance, a wavelength-fixed laser beam source may be used as the laser beam source 61. Or, a wavelength-variable laser beam source which can vary the wavelength of the laser beam may be used as the laser beam source 61. In this case, a laser beam having a proper wavelength can be properly set and irradiated on the basis of the light absorption band of the organic compound or the wavelength of light acting on the solvent.

The laser beam source 61 may be provided with irradiation light intensity adjusting means for adjusting the irradiation light intensity of the laser beam emitted from the laser beam source 61 as occasion demands. An attenuating filter having a high optical pressure resistance, an optical attenuator using light interference/reflection or the like may be used as the irradiation light intensity adjusting means. In the example of Fig. 10, an irradiation light intensity adjuster 61 a such as an attenuating filter or the like is disposed between the laser beam source 61 and the chamber 53. Furthermore, a transmission light intensity measuring device 62 for measuring the transmission light intensity of a laser beam which is emitted from the laser beam source 61 and transmitted through the chamber 53 is disposed at a predetermined position of the opposite side to the laser beam source 61 with respect to the chamber 53.

Furthermore, the fine particle manufacturing apparatus 6 is provided with a monitoring light absorption band measuring device 64 which can measure the light absorption band in the chamber 53. The monitoring light absorption band measuring device 64 has a box for accommodating the chamber 53, and a spectroscopic light source and a photodetector which are provided in the box, and it measures the absorbance of the treatment target liquid 52 in the chamber 53 to monitor the fine pulverization state of the hardly-soluble medicament.

By monitoring the variation of the light absorption band of the treatment target liquid 52 through the monitoring light absorption band measuring device 64 as described above, the fine pulverization state of the medicament 55 is monitored. At this time, it can be referred to when determining an excellent laser beam irradiation time to the treatment target liquid 52 and an irradiation condition, for example, determining stop/continuation of laser beam irradiation in accordance with the fine pulverization state, and it serves to avoid excessive laser beam irradiation to the hardly-soluble medicament 55. Furthermore, the box of the measuring device 64 is provided with a laser beam transmissible port or window through which the laser beam emitted from the laser beam source 61 passes through the chamber 53 and reaches the transmission light intensity measuring device 62. In Fig. 10, the specific construction of the measuring device 64 is omitted from the illustration.

The laser beam source 61, the monitoring light absorption band measuring device 64, the irradiation light intensity adjuster 61a and the transmission light intensity measuring device 62 are electrically connected to a controller 63 comprising a computer or the like. The controller 63 controls the operation of each part of the manufacturing apparatus 6 described above.

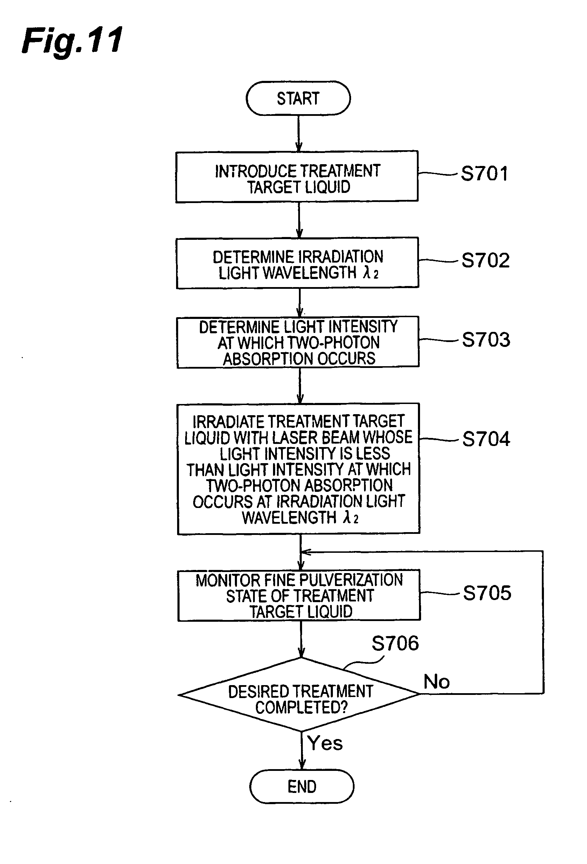

Next, the method for manufacturing fine particles by using the fine particle manufacturing apparatus 6 shown in Fig. 10 will be described with reference to the flowchart of Fig. 11.

First, water 54 and hardly-soluble medicament 55 are mixed, and then stirred to prepare treatment target liquid 52. In the treatment target liquid 52, a part of the hardly-soluble medicament 55 is dissolved in the water 54 to be dissolved material by stirring, and the residual is not dissolved in the water 54 to be non-dissolved material. Subsequently, the treatment target liquid 52 is introduced into the fine particle manufacturing chamber 53 (step S701). The wavelength λ₂ of the laser beam to be irradiated from the laser beam source 61 to the treatment target liquid 52 is set to a wavelength which is different from the light absorption band of the medicament 55 corresponding to an organic compound and acts on the water 54 as solvent (S702). It is preferable that the wavelength of the laser beam is longer than the light absorption band of the medicament 55, and it is more preferable that it is a wavelength of an infrared region.

When the longest wavelength λ₀ of the light absorption band of the medicament 55 as the dissolved material is known, it is preferable that the wavelength λ₂ of the laser beam to be used for the fine particle manufacture is determined by referring to the wavelength λ₀. For example, as the wavelength λ₂ selected is a wavelength which is longer than the longest wavelength λ₀ and acts on the water 54 as solvent.

The laser beam source 61 is controlled by the controller 63, and the wavelength of the laser beam to be irradiated is set at the laser beam source 61 to the laser beam wavelength λ₂ determined as described above. When the wavelength λ₂ is preset, a wavelength-fixed laser beam source for emitting a laser beam having the wavelength λ₂ may be used as the laser beam source 61.

Here, it is preferable that the laser beam irradiation wavelength λ₂ is 900nm or more. Alternatively, it is preferable that the laser beam irradiation wavelength λ₂ is a wavelength of the light absorption band of the solvent. Accordingly, as described later, occurrence of photochemical reactions of the organic compound in the solvent can be reliably prevented while the fine pulverization of the organic compound by the action of the laser beam on the solvent can be sufficiently implemented.

Subsequently, the irradiation light intensity of the laser beam in the fine particle manufacturing process is determined with keeping the laser beam irradiation wavelength λ₂ (S703). First, the laser beam is irradiated to the fine particle manufacturing chamber 53 by the laser beam source 61, and the transmission light intensity of the laser beam transmitted through the fine particle manufacturing chamber 53 is measured by the transmission light intensity measuring device 62. The irradiation light intensity of the laser beam irradiated to the chamber 53 is varied by the irradiation light intensity adjuster 61a while measuring the transmission light intensity of the laser beam transmitted through the fine particle manufacturing chamber 53 by the transmission light intensity measuring device 62, so that the relationship between the irradiation light intensity of the laser beam and the transmission light intensity of the laser beam can be obtained.

Here, when two-photon absorption occurs in the hardly-soluble medicament 55, rapid variation of the transmission light intensity of the laser beam is observed. Therefore, by measuring the relationship between the irradiation light intensity and the transmission light intensity as described above, an irradiation light intensity at which no two-photon absorption occurs in the hardly-soluble medicament 55 can be easily determined. The irradiation light intensity adjuster 61 a is controlled by the controller 63, and the irradiation light intensity of the laser beam is adjusted so as to be smaller than the determined irradiation light intensity at which two-photon absorption occurs.

When a laser beam having an irradiation light intensity at which two-photon absorption occurs in the organic compound is irradiated to the organic compound such as the medicament 55 or the like, a photochemical reaction may occur in the organic compound by two-photon absorption regardless of use of the laser beam having a wavelength at which no photochemical reaction is induced. On the other hand, by irradiating the organic compound with a laser beam having an irradiation light intensity less than the irradiation light intensity at which two-photon absorption occurs, the photochemical reaction in the organic compound can be reliably prevented.

Under this state, the laser beam source 61 is actuated by the controller 63, and the laser beam having the wavelength λ₂ emitted from the laser beam source 61 is irradiated to the fine particle manufacturing chamber 53. Accordingly, the hardly-soluble medicament 55 is finely pulverized in the treatment target liquid 52 in the chamber 53, and fine particles of the hardly-soluble medicament 55 are manufactured (S704).

Here, when the hardly-soluble medicament 55 is a medicinal drug, it is required to avoid excessive laser beam irradiation in the manufacturing process of fine particles. Therefore, the absorbance variation of the treatment target liquid 52 with respect to the laser beam irradiation time is measured for the treatment target liquid 52 by the monitoring light absorption band measuring device 64 to thereby monitor the fine pulverization state, and it is judged whether the desired treatment is completed. When the desired treatment is completed, the laser beam irradiation is stopped. When the desired treatment is not completed, the laser beam irradiation is continued (S705, S706).

Specifically, whether the desired treatment is completed is judged by irradiating a laser beam from the laser beam source 61 to the treatment target liquid 52 and measuring the light absorption band variation by the monitoring light absorption band measuring device 64. When hardly any time variation of the light absorption band is observed, it is judged that the desired treatment is completed. The treatment time may be set to the time period from the start time of the laser beam irradiation untill the time at which the light absorption band hardly varies with respect to the laser beam irradiation time.

The effect of the fine particle manufacturing method and apparatus according to this embodiment will be described.

According to the above fine particle manufacturing method and apparatus, a laser beam having a wavelength which is different from the light absorption band of the organic compound and acts on solvent such as water 54 or the like (preferably, a wavelength absorbed by the solvent) is irradiated to implement the fine pulverization of the organic compound. Accordingly, the organic compound can be finely pulverized into fine particles while more sufficiently preventing occurrence of photochemical reactions of the organic compound in the solvent. Particularly, when only a part of the organic compound is dissolved in the solvent, that is, it is hardly soluble or insoluble in the solvent, the organic compound is finely pulverized by the laser beam irradiation, so that the organic compound can be apparently solubilized in the solvent. Accordingly, liquid containing fine particles of a hardly-soluble or insoluble organic compound can be manufactured.

That is, in the above embodiment, the hardly-soluble medicament 55 is finely pulverized by laser beam irradiation, whereby the hardly-soluble medicament 55 can be apparently solubilized in water 54. Furthermore, even when the hardly-soluble medicament 55 is finely pulverized, the solubility state of the hardly-soluble medicament 55 in water 54 can be kept stable for a long time.

Furthermore, a laser beam having a wavelength different from the light absorption band of the hardly-soluble medicament 55 is used as the laser beam, and the laser beam is not made to directly act on the medicament 55, but made to act on water 54 serving as solvent, thereby finely pulverizing the medicament 55. Accordingly, the fine pulverization of the medicament 55 can be achieved without losing the medicinal benefits while sufficiently preventing occurrence of photochemical reactions of the medicament 55 in water 54.

Furthermore, the fine pulverization treatment can be implemented by only a laser beam having a wavelength which can act on (for example, is absorbed by) solvent such as water 54 or the like irrespective of the light absorption characteristic of the organic compound such as the medicament 55 or the like contained in the treatment target liquid 52. In this case, as compared with the method for setting the laser beam wavelength in connection with the wavelength of the light absorption band of the organic compound, the wavelength of the light source used in the fine particle manufacturing apparatus 6 can be restricted. Accordingly, a laser beam source having a specific wavelength suitable to manufacture fine particles can be developed, and this is effective in the mass treatment and the treatment cost. A semiconductor laser beam source may be used as such a light source. For example, when the solvent is water, a laser beam for emitting a laser beam which has a wavelength of the absorption band of water or a wavelength set on the basis of the wavelength of the absorption band of water can be used irrespective of the organic compound.

With respect to the specific laser beam wavelength, by setting the wavelength to 900nm or more, the fine pulverization treatment of organic compounds can be implemented under the condition that generation of impurities due to photochemical reactions in the organic compounds can be sufficiently suppressed. Furthermore, by setting the laser beam wavelength to a wavelength of the light absorption band of the solvent, the laser beam can be sufficiently absorbed by the solvent and the fine pulverization can be performed with high efficiency.

Even in the case of hardly-soluble material or insoluble material, according to the fine particles of the present invention which are manufactured by the above manufacturing method and apparatus, it can be solubilized in a quasi-style.

It is preferable that water is used as solvent of organic compounds such as medicaments, etc., as described above, or solvent other than water may be used. As such solvent, ethyl alcohol which is monohydric alcohol, glycol group (propylene glycol, polyethylene glycol, etc.) which is dihydric alcohol, glycerol which is trihydric alcohol, etc., may be used. Furthermore, soybean oil, com oil, sesame oil, peanut oil, etc.. which are vegetable oils may be used as solvents. These solvents are suitably used as organic solvents of non-aqueous injectable agents in the cases of injectable agents.

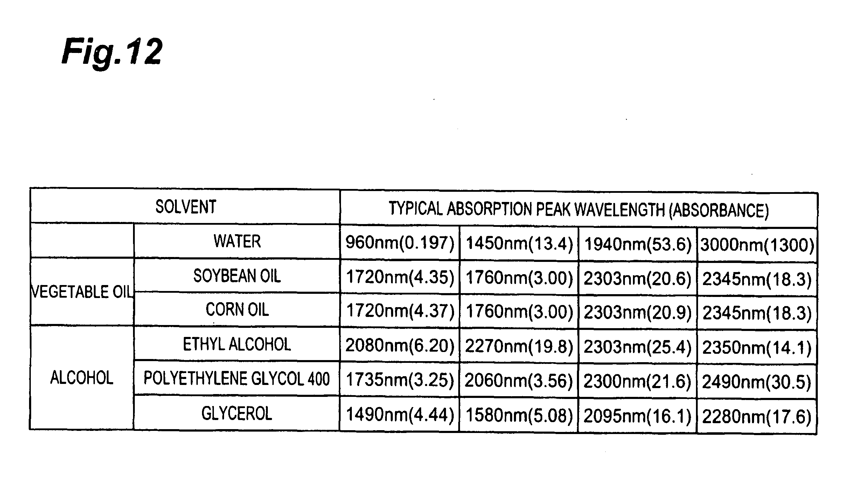

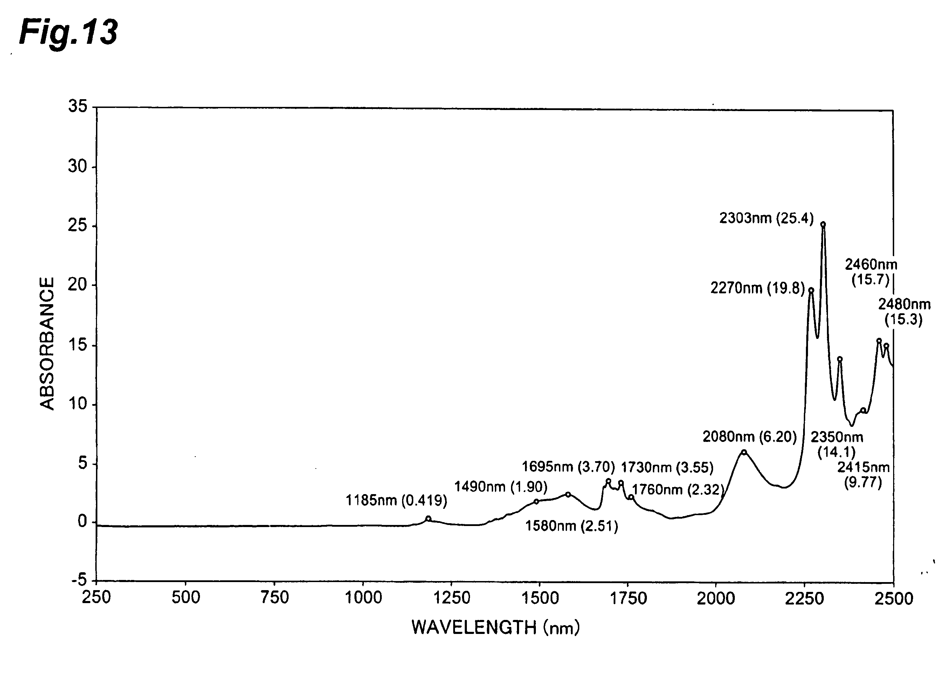

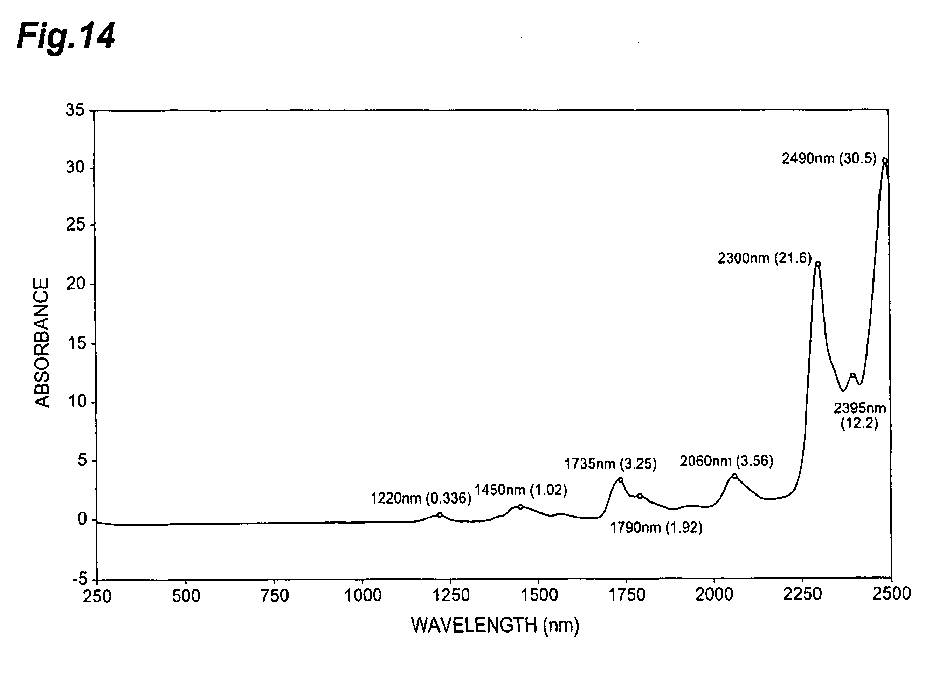

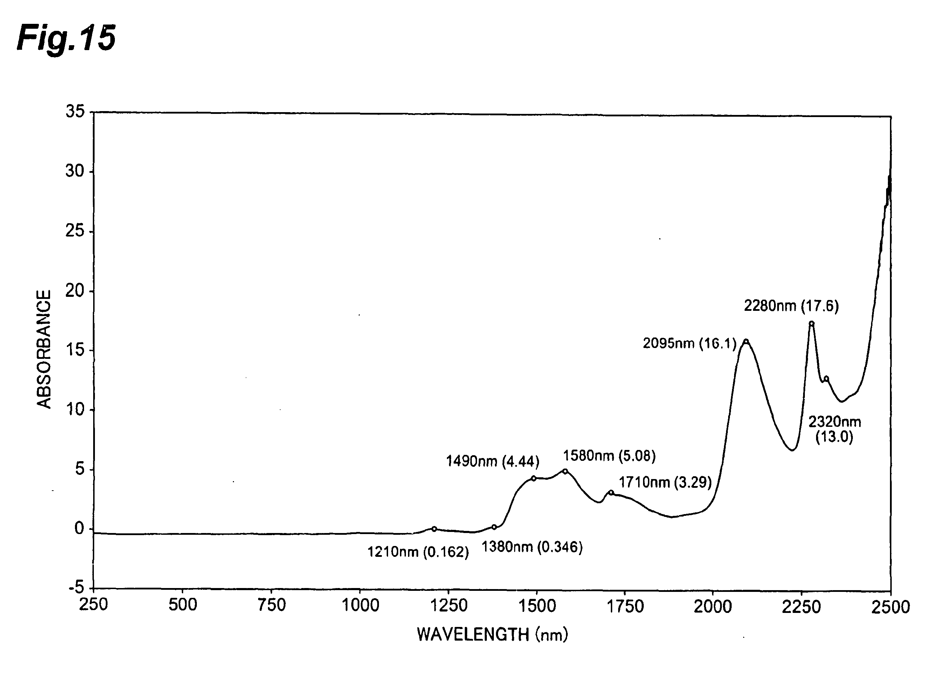

Fig. 12 is a table showing the typical absorption peak wavelengths (nm) and absorbance (a converted value per optical path length of 1 cm is set as absorbance) of the solvents. This table shows the absorption peak wavelengths and absorbance of water, soybean oil, corn oil, ethyl alcohol, polyethylene glycol 400, and glycerol which are solvents permitted to be added as medicinal drugs. Fig. 13, Fig. 14 and Fig. 15 are graphs showing the wavelength dependence of the absorbance of ethyl alcohol, polyethylene glycol 400 and glycerol.

These absorption peak wavelengths are 900nm or more, and the wavelength λ₂ of the laser beam is set to such a peak wavelength or a wavelength in the vicinity of the peak wavelength, whereby the laser beam can be sufficiently absorbed by the solvent and the organic compound in the solvent can be finely pulverized with high efficiency. For example, when water is used as the solvent, the wavelength λ₂ of the laser beam is preferably set to 1450nm, 1940nm or the like.

Here, in the above fine pulverization treatment, it is preferable that the laser beam is irradiated to the treatment target liquid 52 while cooling the treatment target liquid 52, whereby degradation of the organic compound such as the medicament 55 or the like due to thermal decomposition when the laser beam is irradiated can be prevented.

Furthermore, as described above, it is preferable that a laser beam having an irradiation light intensity less than the irradiation light intensity at which two-photon absorption occurs is irradiated to the treatment target liquid 52, whereby the photochemical reactions occurring in the hardly-soluble medicament 55 can be more sufficiently prevented and thus the degeneration of the hardly-soluble medicament 55 can be more sufficiently prevented.

Furthermore, it is preferable that the melting point of the organic compound such as the medicament 55 or the like contained in the treatment target liquid 52 is 250°C or less. Such an organic compound having a low melting point as described above is easily finely pulverized by the action of the laser beam to the solvent. Accordingly, the fine pulverization of the organic compound by the laser beam irradiation can be suitably performed.

That is, according to the method for finely pulverizing the organic compound by the action of the laser beam on the solvent, it is difficult to finely pulverize organic compounds when the organic compounds have strong intermolecular force. On the other hand, organic compounds whose melting point is 250°C or less have relatively weak intermolecular force, and thus they can be suitably finely pulverized by laser beam irradiation.

The fine particles of the hardly-soluble medicament 55 thus achieved not only apparently solubilize in water 54, but also sufficiently keep the medicinal benefits thereof without losing the medicinal benefits thereof. Therefore, there can be performed physical and chemical research and searching and determination of candidate compounds such as screening, etc., the ADME test (absorption, distribution, metabolism, excretory test), general drug toxicity, general pharmacology, pharmacology, biochemical research in preclinical tests of animals, clinical tests, etc., which have not been able to be estimated in conventional manners before the fine pulverization of the hardly-soluble medicament 55 is completed.

Accordingly, even when an achieved compound library or newly synthesized medicaments or natural products are hardly soluble in water, the capital investment thereof is not wasted. Furthermore, the fine particles of the hardly-soluble medicament 55 have a sufficiently large surface area as compared with the state before it is finely pulverized. Accordingly, absorbability by the tissues of living bodies is enhanced, and it can quickly act on the living bodies. Furthermore, according to the fine particle manufacturing method described above, medicaments which can be administered to remarkably various kinds of living bodies can be achieved, and thus the selectivity of medicaments to be administered can be remarkably enlarged. Furthermore, the fine pulverization treatment as described above is also effective for organic compounds other than the medicaments.

In the above manufacturing method, it is preferable that a stabilizer for stably dispersing fine particles of the medicament in the treatment target liquid 52 is added before or during laser beam irradiation. By adding the stabilizer to the treatment target liquid 52 as described above, the hardly-soluble medicament 55 is stably dispersed in water 54 by the stabilizer, and thus the manufacturing efficiency of the fine particles can be enhanced. The stabilizer is preferably a surfactant. In this case, the manufacturing efficiency of the fine particles can be enhanced.

Any material may be used as the stabilizer insofar as it has a property of dispersing the hardly-soluble medicament 55 in water 54 and also has no adverse effect on living bodies. As the stabilizer materials described in the "MEDICINAL ADDITIVE DICTIONARY" or "MEDICINAL ADDITIVE HANDBOOK," may be used, for example, polysorbate group, sorbitane ester group, triethanol amine, cyclodextrin, albumin, etc.

In the above manufacturing method, the absorbance variation of the treatment target liquid 52 in the fine particle manufacturing process is measured by the monitoring light absorption band measuring device 64, and the laser beam irradiation is stopped when the desired treatment is completed. However, the treatment time of the laser beam irradiation may be determined for the same treatment target liquid as the treatment target liquid 52 in advance before fine particles are manufactured. The determination of the treatment time may be carried out, as described above, by measuring the light absorption band of the organic compound by the monitoring light absorption band measuring device and setting the treatment time to the time period from the start time of the laser beam irradiation to the time at which the time-variation of the light absorption band is hardly observed. However, when the treatment time is determined before the fine particles are manufactured, the laser beam irradiation may be stopped in the fine particle manufacturing process at the time when the treatment time has elapsed, and it is not necessary to dispose the monitoring light absorption band measuring device 64 and monitor the fine pulverization state of the medicament in the treatment target liquid 52 by the monitoring light absorption band measuring device 64 during the fine particle manufacturing process.

Next, an embodiment of a method for manufacturing an injectable agent according to the present invention will be described.

First, liquid containing the fine particles of the hardly-soluble medicament 55 which is apparently solubilized in injection water 54 is manufactured by using the fine particle manufacturing apparatus 6 shown in Fig. 10. This liquid manufacturing method is the same as the manufacturing method for the fine particles described above. Stabilizer may be added to the treatment target liquid 52 before or during laser beam irradiation to the hardly-soluble medicament 55 as in the case of the fine particle manufacturing method described above.

Subsequently, an isotonicity agent is added to this liquid to manufacture injectable agent. Here, the isotonicity agent to be added to the liquid has a function of adjusting the osmotic pressure of the injection liquid so that the osmotic pressure of the injection liquid is equal to the blood of a living body. For example, sucrose, physiological salt solution or the like may be used as the isotonicity agent. The fine particles of the hardly-soluble medicament may be manufactured under existence of the isotonicity agent.

According to the manufacturing method described above, the hardly-soluble medicament 55 can be solubilized in the injection water 54 while sufficiently preventing the photochemical reaction thereof. Therefore, even the hardly-soluble medicament 55 can be manufactured as injectable agent. Furthermore, the hardly-soluble medicament 55 is finely pulverized into fine particles, and thus the injectable agent quickly acting on living bodies can be manufactured.

The injectable agent thus manufactured contains medicament fine particles sufficiently keeping the medicinal benefits of the hardly-soluble medicament 55, and thus it can exhibit the same medicinal benefits as the hardly-soluble medicament 55. Furthermore, the surface area of the fine particles is increased by finely pulverizing the hardly-soluble medicament 55, and thus the fine particles thereof have high absorbance to the living bodies. Therefore, the injectable agent has a quick-acting property when it is injected into a living body.

In the above manufacturing apparatus 6, the controller 63 controls the laser beam source 61, the monitoring light absorption band measuring device 64, the irradiation light intensity adjuster 61 a and the transmission light intensity measuring device 62, however, the controller 63 is not necessarily indispensable. Accordingly, an operator may control the laser beam source 61, the monitoring light absorption band measuring device 64, the irradiation light intensity adjuster 61 a and the transmission light intensity measuring device 62.

Furthermore, in the above manufacturing device 6, the material of the fine particle manufacturing chamber 53 is quartz. However, the chamber 53 is not necessarily limited to quartz. However, it is preferable to use as the material of the chamber 53 a material which more greatly absorbs a laser beam having an irradiation light intensity at which two-photon absorption occurs in the hardly-soluble medicament 55 as compared with a laser beam having an irradiation light intensity at which no two-photon absorption occurs. As the material of the chamber 53, not only quartz, but also a chamber made of a semiconductor substrate of silicon or the like, synthesized quartz, glass, polymer (polymer) or the like may be mentioned.

Furthermore, when the light absorption band of the hardly-soluble medicament 55 is measured by using the irradiation wavelength determining light absorption band measuring device, water is preferably used as solvent in the treatment target liquid 52, however, the solvent of the present invention is not limited to water. As the solvent, aqueous organic solvent such as ethyl alcohol or the like, or vegetable oil as described above may be used.

Furthermore, when a medicament is never dissolved in water, that is, it is an insoluble medicament whose light absorption band cannot be measured in water, in order to dissolve a part of the medicament and measure the light absorption band thereof, organic solvent such as ethyl alcohol, acetone, dimethyl sulfoxide or the like or a mixture liquid of the organic solvent concerned and water is used in place of water to separately measure the light absorption band by a spectrophotometer, whereby a proper fine particle manufacturing laser beam irradiation wavelength can be determined.

There is a tendency that when organic solvent is used, the longest wavelength of the light absorption band is shifted as compared with the case where water is used. Therefore, when the light absorption band of the medicament is measured, the mixture of organic solvent and water is preferably used as the solvent. Furthermore, when a laser beam is irradiated to a medicament to manufacture the fine particles thereof, predetermined solvent such as water or the like is required to be used as the solvent from the point of view of preventing an adverse effect on living bodies.

The above embodiment uses as the medicament a hardly-soluble or insoluble medicament such as clobetasone butyrate, carbamazepine or the like, however, the present invention is not limited to these hardly-soluble or insoluble medicaments. Furthermore, in the above embodiment, clobetasone butyrate or carbamazepine which are medicinal drugs are used as medicaments, however, the fine particle manufacturing method and the injection liquid manufacturing method according to the present invention is not limited to the above medicinal drugs, but is applicable to medicinal drug candidate materials (natural products, compound library, etc.), quasi-drugs, cosmetic preparations, etc.

Next, the content of the present invention will be more specifically described on the basis of an example, however, the present invention is not limited to this example.

In this example, fine pulverization of clobetasone butyrate as adrenal cortex hormone of hardly-soluble medicament was attempted. Powder of clobetasone butyrate was suspended at a concentration of 0.5mg/ml in water for ten minutes by using ultrasonic waves, and then 3ml of the suspended liquid (clobetasone butyrate suspended liquid) thus obtained was put in a quartz square cell of 1 cm x 1 cm x 4cm. A stirring magnet stick for stirring the liquid was put in the square cell so that a laser beam was uniformly irradiated to the suspended liquid. The temperature of the suspended liquid was set to room temperature of 25°C at all times except for a temperature dependence test.

In this example, a continuously wavelength-variable OPO parametric oscillator was used as a light source for fine pulverization because the wavelength dependence of the fine pulverization and photochemical reaction by laser beam irradiation was required to be investigated. The pulse width of the irradiation laser beam was set to FWHM 4ns, and the repetition frequency was set to 10Hz.

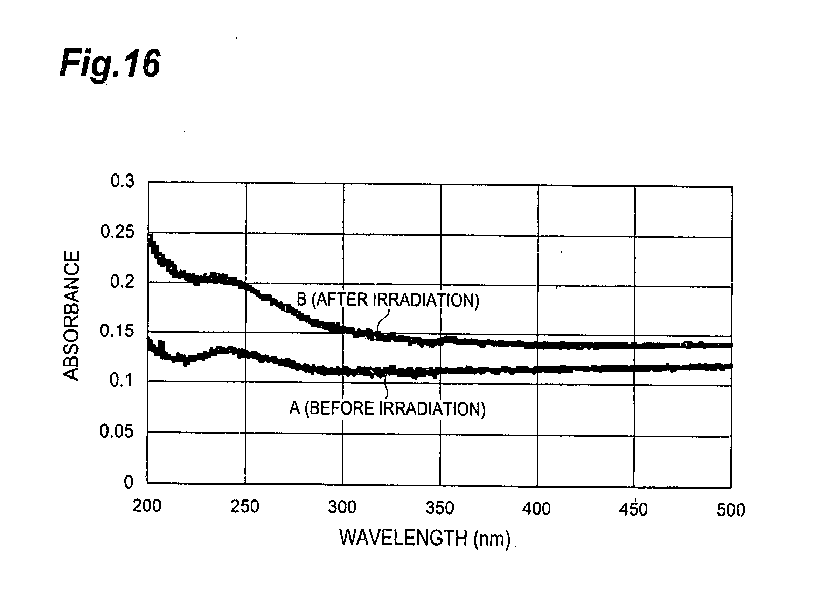

Fig. 16 is a graph showing the wavelength dependence of the absorbance of the clobetasone butyrate suspended liquid before and after the fine pulverization treatment using the above method. In this graph, the abscissa axis represents the wavelength (nm) of light, and the ordinate axis represents the absorbance of the suspended liquid. Graph A represents the light absorption characteristic before the laser beam irradiation, and graph B represents the light absorption characteristic after the laser beam irradiation (after the fine pulverization treatment).

As shown in graph A, in the case of the clobetasone butyrate suspended liquid before the fine pulverization treatment, most of the light absorption characteristic thereof is caused by light scattering loss, and it is a flat light absorption characteristic having small wavelength dependence. A YAG pulse laser beam of 1700mJ/cm² in irradiation light intensity per pulse and 1064nm in wavelength was irradiated to the suspended liquid for one hour to finely pulverize clobetasone butyrate. After the irradiation treatment described above, as shown in graph B, the light absorption characteristic of clobetasone butyrate itself appeared. This phenomenon shows that the fine pulverization of the suspended liquid progressed untill the sub-micrometer order.

Subsequently, the irradiation wavelength of the laser beam was varied and the test was carried out. In addition, the purity of clobetasone butyrate after the fine pulverization treatment based on the laser beam irradiation (use of the lowest purity of 98%, produced by SIGMA) was measured by using a high performance liquid chromatography (HPLC), and the laser beam wavelength dependence was investigated. The laser beam irradiation intensity at each wavelength was set to such a level that the fine pulverization of the sub-micrometer order could be observed as shown in Fig. 16, and the laser beam irradiation treatment was carried out for one hour.

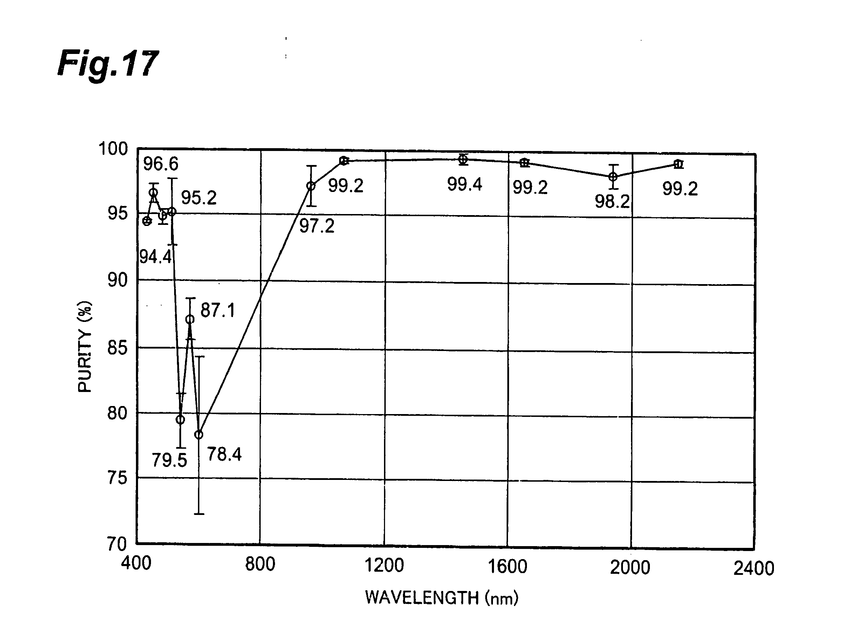

Fig. 17 is a graph showing the laser beam wavelength dependence of the purity of clobetasone butyrate after the fine pulverization treatment. In this graph, the abscissa axis represents the wavelength (nm) of the laser beam irradiated to the suspended liquid, and the ordinate axis represents the clobetasone butyrate purity (%).

When the wavelength of the laser beam to be irradiated ranges from 500 to 800nm, it is impossible to finely pulverize clobetasone butyrate unless the laser beam has a light intensity of very high output. Therefore, as shown in the graph, a part of clobetasone butyrate induces a photochemical reaction, and great degradation of the purity was observed. Furthermore, at the wavelength of 500nm or less, the light intensity needed for the fine pulverization treatment is relatively small, and thus the degradation is also small. However, energy per photon is large, and clobetasone butyrate directly absorbs the laser beam, so that a photochemical reaction likewise occurs. As a result, degradation occurs and a generation rate of impurities which is permitted for the medicament treatment is not satisfied.

On the other hand, as shown in Fig. 17, by carrying out the fine pulverization treatment with an infrared laser beam of 900nm or more in wavelength, it is possible to carry out the fine pulverization treatment under a condition that the photochemical reaction hardly occurs in the organic compound such as a medicament or the like. If the light intensity is excessively high, degradation of the purity by thermal decomposition may be considered. In such a case, it is preferable that the temperature of the treatment target liquid is reduced by cooling the treatment target liquid.

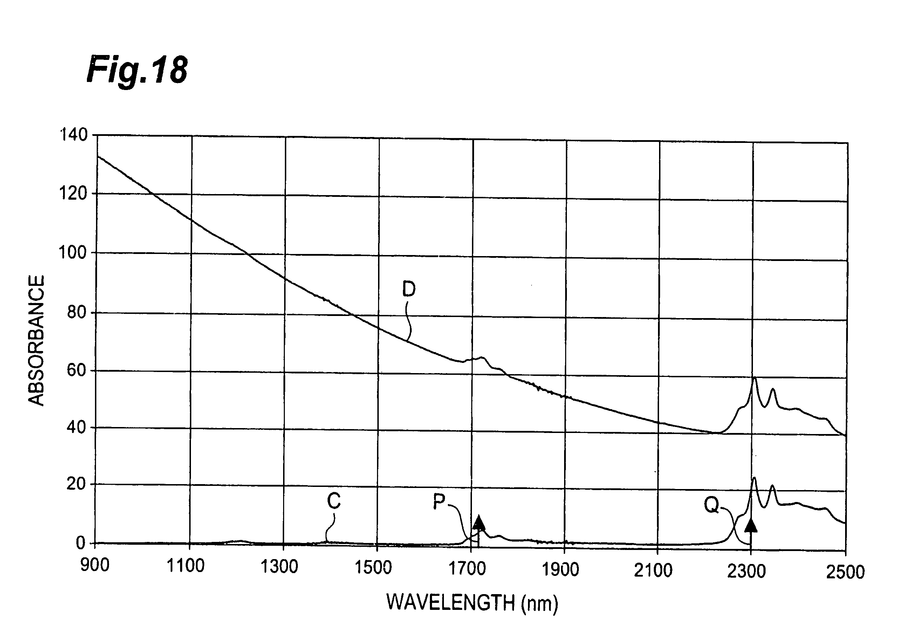

Fig. 18 is a graph showing the light absorption characteristic of clobetasone butyrate in the infrared wavelength region. In this graph, the abscissa axis represents the wavelength (nm) of light, and the ordinate axis represents the absorbance of clobetasone butyrate. Graph C represents the light absorption characteristic in the case of only liquid paraffin, and graph D represents the light absorption characteristic in the case of the mixture liquid of clobetasone butyrate and liquid paraffin.

In this case, the light absorption characteristic of clobetasone butyrate in the infrared wavelength region was measured by using the Nujol method. The Nujol method is a method for adding liquid paraffin to a particle-state sample, pounding the sample in a mortar to process the samples like oil, and estimating the light absorption characteristic of the sample itself from the difference in light absorption characteristic between the mixture liquid and the liquid paraffin. In the measurement of the absorbance, a quartz cell having an optical path length of 100µm was used.

As shown in the graph D, light scattering and a slight light absorption band appear in the light absorption characteristic of the mixture liquid of clobetasone butyrate and liquid paraffin. Comparing this light absorption characteristic with the light absorption characteristic of only the liquid paraffin shown in the graph C, both the light absorption bands of a 1700nm band and a 2300nm band indicated by arrows P and Q are coincident with each other. From this, it is considered that clobetasone butyrate itself has no great light absorption band at the wavelength area from 900 to 2500nm, and thus occurrence of the photochemical reaction in clobetasone butyrate is sufficiently small even when a laser beam in this wavelength range is irradiated for the fine pulverization treatment.

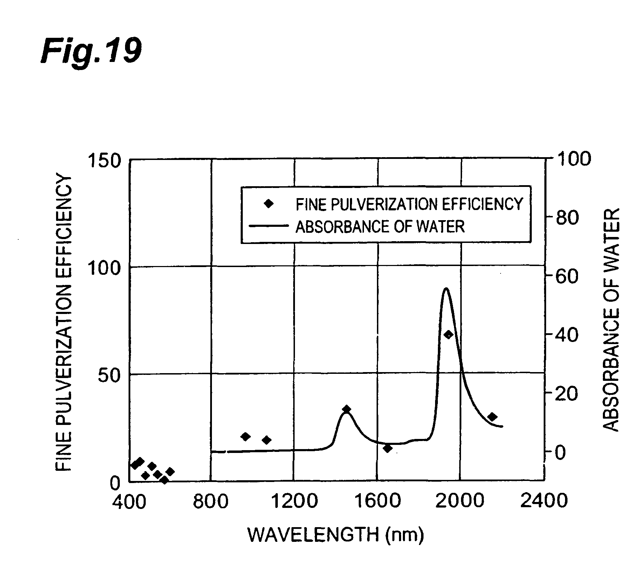

Next, the fine pulverization efficiency was determined in the wavelength range of 420 to 2150nm in the laser beam wavelength. Fig. 19 is a graph showing the laser beam wavelength dependence of the fine pulverization efficiency. In this graph, the abscissa axis represents the wavelength (nm) of the laser beam, and the ordinate axis represents the fine pulverization efficiency which is normalized with the fine pulverization efficiency at a wavelength of 570nm being set to 1.

Here, as a method for calculating the fine pulverization efficiency, the absorbance of clobetasone butyrate which indicates the degree of fine pulverization is determined, divided by the irradiation light intensity, and normalized by the fine pulverization efficiency at the wavelength of 570nm to compare the fine pulverization efficiencies at the respective wavelengths. In Fig. 19, the graph of the wavelength dependence of the absorbance of water is shown in association with the data of the fine pulverization efficiency.

As shown in this graph, when the wavelength of the laser beam to be irradiated is 570nm, the fine pulverization efficiency is lowest, and there is little difference even at wavelengths of 420 to 600nm. On the other hand, the fine pulverization is efficiently performed at wavelengths of 900nm or more. Furthermore, water has light absorption bands at 960nm, 1450nm and 1940nm, and it has been clarified that the fine pulverization efficiency is particularly enhanced at absorption wavelengths of water. This shows that the fine pulverization treatment based on the laser beam irradiation could be performed if a wavelength is selected at which no light absorption occurs in organic compounds such as clobetasone butyrate, etc., in the near-infrared wavelength area, but which acts on (absorption occurs) solvent such as water or the like.

The fine particles, the manufacturing method and apparatus thereof, and the injectable agent and the manufacturing method thereof according to the present invention are not limited to the above embodiments and examples, and various modifications may be performed.

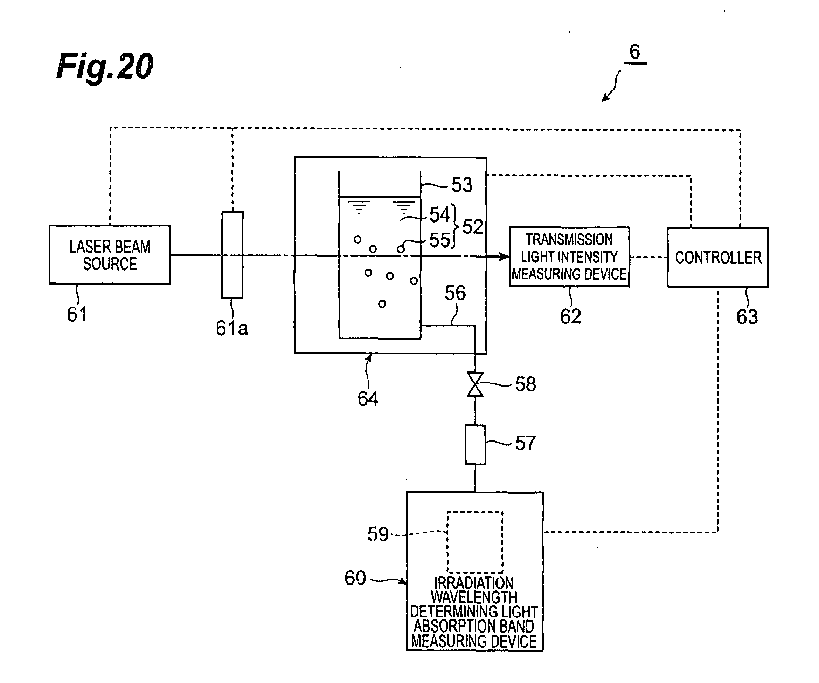

Fig. 20 is a block diagram showing a modified example of the fine particle manufacturing apparatus shown in Fig. 10. In the fine particle manufacturing apparatus 6 of this modification, the chamber 53 for accommodating the treatment target liquid 52 comprising water 54 and a hardly-soluble medicament 55, the laser beam source 61, the irradiation light intensity adjuster 61a, the transmission light intensity measuring device 62, the controller 63 and the monitoring light absorption band measuring device 64 have the same constructions as shown in Fig. 10.

In this configuration example, a drain pipe 56 for draining the treatment target liquid 52 from the chamber 53 is connected to the lower side of the chamber 53. In the drain pipe 56 are disposed a valve 58 and a separation filter 57 for transmitting therethrough the treatment target liquid 52 discharged from the chamber 53 and separating the non-dissolved material of the hardly-soluble medicament 55 from the treatment target liquid 52. Furthermore, the fine particle manufacturing apparatus 6 is provided with irradiation wavelength determining light absorption band measuring device 60 containing a light absorption band analyzing chamber 59.

The drain pipe 56 is connected to the light absorption band analyzing chamber 59 of the irradiation wavelength determining light absorption band measuring device 60. Accordingly, when the valve 58 is opened, a part of the treatment target liquid 52 in the fine particle manufacturing chamber 53 is drained from the chamber 53 through the drain pipe 56, and the non-dissolved material (solid material) of the hardly-soluble medicament 55 is separated from the treatment target liquid 52 by the separation filter 57. The treatment target liquid 52 containing the dissolved material which transmitted through the separation filter 57 is introduced into the light absorption band analyzing chamber 59, and the light absorption band of the dissolved material dissolved in the water 54 is measured by the irradiation wavelength determining light absorption band measuring device 60.

The manufacturing apparatus 6 is provided with the irradiation wavelength determining light absorption band measuring device 60 as described above, whereby the light absorption band of the hardly-soluble medicament 55 can be immediately measured by introducing the treatment target liquid 52 discharged from the chamber 53 to the light absorption analyzing chamber 59 even when the light absorption band of the hardly-soluble medicament 55 is unknown. The light absorption band thus measured, for example, can be referred to when the wavelength of the laser beam to be irradiated to the treatment target liquid 52 from the laser beam source 61 is determined.

Furthermore, the non-dissolved material can be reliably removed from the treatment target liquid 52 introduced to the light absorption band analyzing chamber 59 by the separation filter 57, and thus the light absorption band of the dissolved material can be accurately measured. The drain pipe 56, the separation filter 57, the valve 58 and the light absorption band measuring device 60 constitute the irradiation wavelength determining light absorption band measuring means. The light absorption band measuring means may be eliminated as shown in Fig. 10 as occasion demands, for example, when the light absorption band of the medicament 55 is known or when the wavelength of the laser beam is preset.

The irradiation wavelength determining light absorption band measuring device 60, the laser beam source 61, the monitoring light absorption band measuring device 64, the irradiation light intensity adjuster 61 a and the transmission light intensity measuring device 62 are electrically connected to a controller 63 such as a computer. The controller 63 controls the operations of the respective parts of the manufacturing apparatus 6.

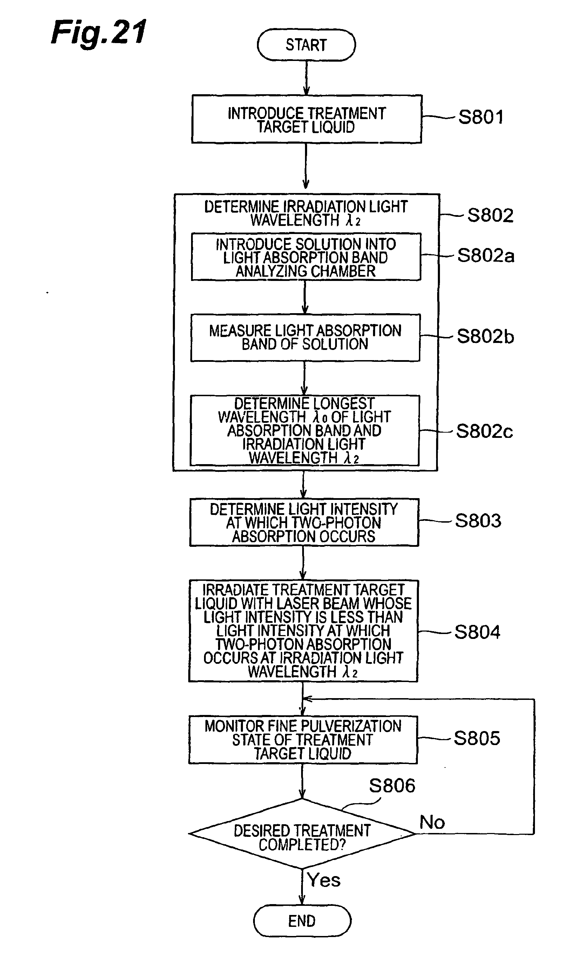

Fig. 21 is a flowchart showing the fine particle manufacturing method using the fine particle manufacturing apparatus 6 shown in Fig. 20. Steps S801 to S806 in the manufacturing method shown in Fig. 21 are the same as the steps S701 to S706 in the manufacturing method shown in Fig. 11, however, are different in that the light absorption band of solution is measured in the step S802 for determining the irradiation light wavelength λ₂.

That is, in the manufacturing apparatus 6 shown in Fig. 20, the following measurement of the light absorption band may be carried out on the treatment target liquid 52 containing the medicament 55 to be finely pulverized in order to set the wavelength of the laser beam as occasion demands. First, the valve 58 disposed in the drain pipe 56 is opened by the controller 63, and a part of the treatment target liquid 52 is drained from the chamber 53 to the drain pipe 56. In the separation filter 57, the non-dissolved material of the hardly-soluble medicament 55 is separated from the treatment target liquid 52, and the residual is introduced as the solution into the light absorption band analyzing chamber 59 (S802a).

Subsequently, with respect to the dissolved material of the hardly-soluble medicament 55 in the solution introduced into the light absorption band analyzing chamber 59, the light absorption band is measured by the light absorption band measuring device 60 (S802b). The measurement result of the light absorption band is transmitted to the controller 63, and the longest wavelength λ₀ is determined on the basis of the measurement result of the light absorption band for the dissolved material in the controller 63. Here, the longest wavelength λ₀ of the light absorption band is defined as a wavelength at the base of the mountain at the long wavelength side of the light absorption band in the absorbance characteristic, at which variation of the absorbance that clearly seems to be caused by electron transition absorption of the dissolved material can be confirmed as compared with the absorbance in a longer wavelength area.

After the longest wavelength λ₀ of the light absorption band of the medicament 55 serving as the dissolved material is determined as described above, the wavelength λ₂ of the laser beam used for the manufacture of fine particles is determined by referring to the wavelength λ₀. For example, the wavelength λ₂ is set as a wavelength which is longer than the longest wavelength λ₀ and acts on water 54 of the solvent (S802c). The laser beam source 61 is controlled by the controller 63, and the laser beam wavelength to be irradiated is set to the laser beam wavelength λ₂ thus determined in the laser beam source 61.

With respect to the setting of the wavelength λ₂, as shown in Fig. 11, the wavelength λ₂ may be set without carrying out the steps S802a to S802c shown in Fig. 21 in such a case that the light absorption band of the medicament 55 is known in advance. Furthermore, when the wavelength λ₂ is preset, a wavelength-fixed laser beam source for emitting a laser beam having the wavelength λ₂ may be used as the laser beam source 61.

### Industrial Applicability

According to the fine particle manufacturing method and apparatus of the present invention as described above, a laser beam having a wavelength longer than the light absorption band of an organic compound is used, and thus even when the laser beam is irradiated to the organic compound, the fine particles of the organic compound can be manufactured with sufficiently preventing occurrence of photochemical reactions in the organic compound.

According to the fine particles of the present invention, even in the case of hardly-soluble material or insoluble material, it can be solubilized in a quasi-style.

According to the injectable agent of the present invention, when it is injected into a living body, it quickly acts on the living body.

Furthermore, according to the manufacturing method for the injectable agent according to the present invention, even when it is a medicament which is insoluble or partially dissolved in water, it can be manufactured as injectable agent. Furthermore, injectable agent that quickly acts on living bodies can be manufactured.

Furthermore, according to the method and apparatus of irradiating the treatment target liquid with a laser beam having a predetermined wavelength which is different from the light absorption band of the organic compound and acts on the solvent when the organic compound in the solvent of the treatment target liquid is finely pulverized to manufacture fine particles of the organic compound, the fine pulverization of the organic compound can be implemented by irradiating the light having the predetermined wavelength acting on the solvent irrespective of the light absorption characteristic of the organic compound contained in the treatment target liquid. Accordingly, the fine pulverization of the organic compound can be performed with sufficiently preventing occurrence of photochemical reactions of the organic compound in the solvent.

Furthermore, according to the fine particles of the present invention, even in the case of hardly-soluble material or insoluble material, it can be finely pulverized and apparently solubilized. Furthermore, according to the injectable agent of the present invention, when it is injected into a living body, it quickly acts on the living body. Furthermore, according to the manufacturing method for the injectable agent of the present invention, even when medicament is insoluble or partially dissolved in water, it can be manufactured as injectable agent. Furthermore, injectable agent that quickly acts on living bodies can be manufactured.

## Claims

1. A fine particle manufacturing method in which an organic compound in solvent of treatment target liquid is finely pulverized to manufacture a fine particle of the organic compound, comprising:
a preparation step of preparing treatment target liquid containing an organic compound and solvent mixed with each other; and
a laser beam irradiation step of irradiating a laser beam having a wavelength longer than the light absorption band of the organic compound to the treatment target liquid to finely pulverize the organic compound into a fine particle.
wherein the organic compound is a medicament; and
wherein the irradiation light intensity of the laser beam to the treatment target liquid is set to less than the irradiation light intensity at which two-photon absorption occurs in the organic compound.

2. The manufacturing method according to Claim 1, wherein only a part of the organic compound is dissolved in the solvent.

3. The manufacturing method according to Claim 1 or 2, wherein the organic compound is insoluble in the solvent.

4. The manufacturing method according to any one of Claims 1 to 3, wherein during the irradiation of the laser beam to the treatment target liquid, the absorbance of the organic compound in the treatment target liquid is measured to monitor a fine pulverization state of the organic compound.

5. The manufacturing method according to any one of Claims 1 to 4, wherein the irradiation light intensity of the laser beam irradiated to a chamber is varied while measuring the transmission light intensity of the laser beam transmitted through the treatment target liquid in the chamber, thereby determining the irradiation light intensity at which two-photon absorption occurs in the organic compound.

6. The manufacturing method according to any one of Claims 1 to 5, wherein a stabilizer for stably dispersing fine particles manufactured in the treatment target liquid is added to the treatment target liquid before or during the irradiation of the laser beam to the treatment target liquid.

7. The manufacturing method according to Claim 6, wherein the stabilizer is surfactant.

8. The manufacturing method according to Claim 7, wherein after the surfactant is added to the treatment target liquid, the treatment target liquid is diluted to separate the fine particles from the surfactant, thereby obtaining an aggregating fine particle which is an aggregate of the fine particles.

9. The manufacturing method according to any one of Claims 1 to 8, wherein in the laser beam irradiation step, a laser beam having a predetermined wavelength which is different form the light absorption band of the organic compound and acts on the solvent is irradiated to the treatment target liquid as the laser beam having the wavelength longer than the light absorption band of the organic compound.

10. The manufacturing method according to Claim 9, wherein the wavelength of the laser beam is a wavelength of the light absorption band of the solvent.

11. The manufacturing method according to any one of Claims 1 to 10, wherein the wavelength of the laser beam is 900nm or more.

12. The manufacturing method according to any one of Claims 1 to 11, wherein the laser beam is irradiated to the treatment target liquid while cooling the treatment target liquid.

13. The manufacturing method according to any one of Claims 1 to 12, wherein the melting point of the organic compound is 250°C or less.

14. A fine particle manufacturing apparatus for finely pulverizing an organic compound in solvent of treatment target liquid to manufacture a fine particle of the organic compound, comprising:
a chamber for accommodating treatment target liquid containing an organic compound having a prescribed light absorption band and solvent mixed with each other; and
a laser beam source for irradiating a laser beam having a wavelength longer than the light absorption band of the organic compound to the treatment target liquid accommodated in the chamber,
wherein the organic compound is a medicament, and
the manufacturing apparatus further comprising:
irradiation light intensity adjusting means for adjusting the irradiation light intensity of the laser beam to be irradiated to the chamber from the laser beam source; and
control means for controlling the irradiation light intensity adjusting means so that the irradiation light intensity of the laser beam to the treatment target liquid is adjusted to less than the irradiation light intensity at which two-photon absorption occurs in the organic compound.

15. The manufacturing apparatus according to Claim 14, wherein the laser beam source is a wavelength-variable laser.

16. The manufacturing apparatus according to Claim 15, further comprising irradiation wavelength determining light absorption band measuring means for discharging a part of the treatment target liquid from the chamber, and measuring the absorbance of the organic compound in the treatment target liquid to determine the wavelength of the laser beam to be irradiated to the organic compound;
wherein the irradiation wavelength determining light absorption band measuring means has a separation filter for separating a solid material from the treatment target liquid discharged from the chamber, and the absorbance of the organic compound in the treatment target liquid from which the solid material is separated by the separation filter is measured.

17. The manufacturing apparatus according to any one of Claims 14 to 16, further comprising:
a transmission light intensity measuring device for measuring the transmission light intensity of the laser beam transmitted through the treatment target liquid in the chamber.

18. The manufacturing apparatus according to Claim 17, wherein the chamber absorbs more greatly a laser beam that has a wavelength longer than the light absorption band and has an irradiation light intensity at which two-photon absorption occurs in the organic compound, as compared with a laser beam having an irradiation light intensity at which no two-photon absorption occurs.

19. The manufacturing apparatus according to any one of Claims 14 to 18, wherein the laser beam source irradiates the treatment target liquid accommodated in the chamber with a laser beam having a predetermined wavelength that is different from the light absorption band of the organic compound and acts on the solvent, as the laser beam having a wavelength longer than the light absorption band of the organic compound.

20. The manufacturing apparatus according to Claim 19, wherein the wavelength of the laser beam is a wavelength of the light absorption band of the solvent.

21. The manufacturing apparatus according to any one of Claims 14 to 20, wherein the wavelength of the laser beam is set to 900nm or more.

22. The manufacturing apparatus according to any one of Claims 14 to 21, further comprising monitoring light absorption band measuring means for measuring the absorbance of the organic compound in the treatment target liquid to monitor a fine pulverization state of the organic compound.

23. A medicament in fine particle form manufactured by the fine particle manufacturing method according to any one of Claims 1 to 13.

24. An injectable agent manufacturing method, wherein liquid containing fine particles is manufactured by the fine particle manufacturing method according to Claim 1, and an isotonicity agent is added to the liquid to manufacture the injectable agent.

25. Injectable agent manufactured by the injectable agent manufacturing method according to Claim 24.

## Patentansprüche

1. Verfahren zur Herstellung feiner Teilchen, bei dem eine organische Verbindung in Lösungsmittel einer zu behandelnden Flüssigkeit fein pulverisiert wird, um feine Teilchen der organischen Verbindung herzustellen, umfassend:
einen Vorbereitungsschritt, bei dem die zu behandelnde Flüssigkeit, die eine organische Verbindung und Lösungsmittel miteinander gemischt enthält, herzustellen; und
einen Laserstrahl-Bestrahlungsschritt, bei dem die zu behandelnde Flüssigkeit mit einem Laserstrahl, der eine Wellenlänge hat, die länger als die Lichtabsorptionsbande der organischen Verbindung ist, bestrahlt wird, um die organische Verbindung in feine Teilchen zu pulverisieren;
wobei die organische Verbindung ein Medikament ist; und
wobei die Bestrahlungs-Lichtintensität des Laserstrahls für die zu behandelnde Flüssigkeit auf weniger als die Bestrahlungs-Lichtintensität eingestellt wird, bei der Zwei-Photonen-Absorption in der organischen Verbindung auftritt.

2. Herstellungsverfahren nach Anspruch 1, wobei nur ein Teil der organischen Verbindung in dem Lösungsmittel gelöst ist.

3. Herstellungsverfahren nach Anspruch 1 oder 2, wobei die organische Verbindung in dem Lösungsmittel unlöslich ist.

4. Herstellungsverfahren nach einem der Ansprüche 1 bis 3, wobei während der Bestrahlung der zu behandelnden Flüssigkeit mit dem Laserstrahl die Absorption der organischen Verbindung in der zu behandelnden Flüssigkeit gemessen wird, um den Feinpulverisierungszustand der organischen Verbindung zu überwachen.

5. Herstellungsverfahren nach einem der Ansprüche 1 bis 4, wobei die Bestrahlungs-Lichtintensität des Laserstrahls, mit dem eine Kammer bestrahlt wird, verändert wird, während die Transmissions-Lichtintensität des Laserstrahls, der durch die zu behandelnde Flüssigkeit in der Kammer transmittiert wird, gemessen wird, wodurch die Bestrahlungs-Lichtintensität, bei der Zwei-Photonen-Absorption in der organischen Verbindung auftritt, bestimmt wird.

6. Herstellungsverfahren nach einem der Ansprüche 1 bis 5, wobei ein Stabilisierungsmittel, um die in der zu behandelnden Flüssigkeit hergestellten feinen Teilchen dauerhaft zu dispergieren, vor oder während der Bestrahlung der zu behandelnden Flüssigkeit mit dem Laserstrahl zugegeben wird.

7. Herstellungsverfahren nach Anspruch 6, wobei das Stabilisierungsmittel ein Tensid ist.

8. Herstellungsverfahren nach Anspruch 7, wobei die zu behandelnde Flüssigkeit, nachdem das Tensid zu der zu behandelnden Flüssigkeit gegeben wurde, verdünnt wird, um die feinen Teilchen von dem Tensid abzutrennen, wodurch Aggregieren der feinen Teilchen erreicht wird, was ein Aggregat der feinen Teilchen ist.

9. Herstellungsverfahren nach einem der Ansprüche 1 bis 8, wobei die zu behandelnde Flüssigkeit bei dem Laserstrahl-Bestrahlungsschritt mit einem Laserstrahl, der eine festgesetzte Wellenlänge hat, die von der Lichtabsorptionsbande der organischen Verbindung verschieden ist, und der auf das Lösungsmittel wirkt, bestrahlt wird, wobei der Laserstrahl eine längere Wellenlänge hat als die Lichtabsorptionsbande der organischen Verbindung.

10. Herstellungsverfahren nach Anspruch 9, wobei die Wellenlänge des Laserstrahls eine Wellenlänge der Lichtabsorptionsbande des Lösungsmittels ist.

11. Herstellungsverfahren nach einem der Ansprüche 1 bis 10, wobei die Wellenlänge des Laserstrahls 900 nm oder mehr ist.

12. Herstellungsverfahren nach einem der Ansprüche 1 bis 11, wobei die zu behandelnde Flüssigkeit mit dem Laserstrahl bestrahlt wird, während die zu behandelnde Flüssigkeit gekühlt wird.

13. Herstellungsverfahren nach einem der Ansprüche 1 bis 12, wobei der Schmelzpunkt der organischen Verbindung 250°C oder weniger ist.

14. Vorrichtung zur Herstellung feiner Teilchen, um eine organische Verbindung in Lösungsmittel einer zu behandelnden Flüssigkeit fein zu pulverisieren, um feine Teilchen der organischen Verbindung herzustellen, umfassend:
eine Kammer, um die zu behandelnde Flüssigkeit aufzunehmen, die eine organische Verbindung, die eine festgesetzte Lichtabsorptionsbande hat, und ein Lösungsmittel miteinander gemischt enthält; und
eine Laserstrahlquelle, um die in der Kammer aufgenommene zu behandelnde Flüssigkeit mit einem Laserstrahl zu bestrahlen, der eine längere Wellenlänge hat als die Lichtabsorptionsbande der organischen Verbindung,
wobei die organische Verbindung ein Medikament ist, und
die Herstellungsvorrichtung weiterhin umfasst:
ein Justierungsmittel für die Bestrahlungs-Lichtintensität, um die Bestrahlungs-Lichtintensität des Laserstrahls, mit dem die Kammer aus der Laserstrahlquelle bestrahlt werden soll, einzustellen; und
ein Kontrollmittel, um das Justierungsmittel für die Bestrahlungs-Lichtintensität zu kontrollieren, so dass die Bestrahlungs-Lichtintensität des Laserstrahls für die zu behandelnde Flüssigkeit auf weniger als die Bestrahlungs-Lichtintensität bei der Zwei-Photonen-Absorption in der organischen Verbindung auftritt, eingestellt wird.

15. Herstellungsvorrichtung nach Anspruch 14, wobei die Laserstrahlquelle ein Laser mit variabler Wellenlänge ist.

16. Herstellungsvorrichtung nach Anspruch 15, weiterhin umfassend
ein Messgerät zur Bestimmung der Lichtabsorptionsbande der Bestrahlungs-Wellenlänge, um einen Teil der zu behandelnden Flüssigkeit aus der Kammer auszulassen und die Absorption der organischen Verbindung in der zu behandelnden Flüssigkeit zu messen, um die Wellenlänge des Laserstrahls, mit dem die organische Verbindung bestrahlt werden soll, zu bestimmen;
wobei das Messgerät zur Bestimmung der Lichtabsorptionsbande der Bestrahlungs-Wellenlänge einen Trennungsfilter besitzt, um festes Material aus der zu behandelnden Flüssigkeit, die aus der Kammer ausgelassen wurde, zu entfernen und die Absorption der organischen Verbindung in der zu behandelnden Flüssigkeit, aus der das feste Material durch den Trennungsfilter abgetrennt wurde, gemessen wird.

17. Herstellungsvorrichtung nach einem der Ansprüche 14 bis 16, weiterhin umfassend:
ein Messgerät für die Transmissions-Lichtintensität, um die Transmissions-Lichtintensität des Laserstrahls, der durch die zu behandelnde Flüssigkeit in der Kammer transmittiert wird, zu messen.

18. Herstellungsvorrichtung nach Anspruch 17, wobei die Kammer einen Laserstrahl, der eine Wellenlänge hat, die länger als die Lichtabsorptionsbande ist und der eine Bestrahlungs-Lichtintensität hat, bei der Zwei-Photonen-Absorption in der organischen Verbindung auftritt, stärker absorbiert als einen Laserstrahl, der eine Bestrahlungs-Lichtintensität hat, bei der keine Zwei-Photonen-Absorption auftritt.

19. Herstellungsvorrichtung nach einem der Ansprüche 14 bis 18, wobei die Laserstrahlquelle die in der Kammer aufgenommene zu behandelnde Flüssigkeit mit einem Laserstrahl bestrahlt, der eine festgesetzte Wellenlänge hat, die von der Lichtabsorptionsbande der organischen Verbindung verschieden ist, und der auf das Lösungsmittel wirkt, wobei der Laserstrahl eine längere Wellenlänge hat als die Lichtabsorptionsbande der organischen Verbindung.

20. Herstellungsvorrichtung nach Anspruch 19, wobei die Wellenlänge des Laserstrahls eine Wellenlänge der Lichtabsorptionsbande des Lösungsmittels ist.

21. Herstellungsvorrichtung nach einem der Ansprüche 14 bis 20, wobei die Wellenlänge des Laserstrahls auf 900 nm oder mehr eingestellt ist.

22. Herstellungsvorrichtung nach einem der Ansprüche 14 bis 21, weiterhin umfassend ein Messgerät für die Überwachung der Lichtabsorptionsbande, um die Absorption der organischen Verbindung in der zu behandelnden Flüssigkeit zu messen, um den Feinpulverisierungszustand der organischen Verbindung zu überwachen.

23. Medikament in Form feiner Teilchen, hergestellt durch das Verfahren zur Herstellung feiner Teilchen nach einem der Ansprüche 1 bis 13.

24. Verfahren zur Herstellung eines injizierbaren Mittels, wobei Flüssigkeit, die feine Teilchen enthält, durch das Verfahren zur Herstellung feiner Teilchen nach Anspruch 1 hergestellt wird und ein Isotonizitätsmittel zu der Flüssigkeit gegeben wird, um das injizierbare Mittel herzustellen.

25. Injizierbares Mittel, hergestellt durch das Verfahren zur Herstellung eines injizierbaren Mittels nach Anspruch 24.

## Revendications

1. Procédé de fabrication de particules fines dans lequel un composé organique dans un solvant d'un liquide cible de traitement est finement pulvérisé pour fabriquer une particule fine du composé organique, comprenant :
une étape de préparation consistant à préparer un liquide cible de traitement contenant un composé organique et un solvant mélangés entre eux ; et
une étape d'irradiation par faisceau laser consistant à irradier par un faisceau laser, ayant une longueur d'onde plus grande que la bande d'absorption de lumière du composé organique, le liquide cible de traitement pour pulvériser finement le composé organique en une fine particule,
dans lequel le composé organique est un médicament ; et
dans lequel l'intensité de la lumière d'irradiation du faisceau laser sur le liquide cible de traitement est réglée de façon à être inférieure à l'intensité de la lumière d'irradiation à laquelle une absorption de deux photons a lieu dans le composé organique.

2. Procédé de fabrication selon la revendication 1, dans lequel seule une partie du composé organique est dissoute dans le solvant.

3. Procédé de fabrication selon la revendication 1 ou 2, dans lequel le composé organique est insoluble dans le solvant.

4. Procédé de fabrication selon l'une quelconque des revendications 1 à 3, dans lequel, pendant l'irradiation par le faisceau laser du liquide cible de traitement, l'absorbance du composé organique dans le liquide cible de traitement est mesurée pour contrôler un état de pulvérisation fine du composé organique.

5. Procédé de fabrication selon l'une quelconque des revendications 1 à 4, dans lequel on fait varier l'intensité de la lumière d'irradiation du faisceau laser irradiant une chambre tout en mesurant l'intensité de la lumière de transmission du faisceau laser transmise à travers le liquide cible de traitement dans la chambre, de manière à déterminer l'intensité de la lumière d'irradiation à laquelle une absorption de deux photons a lieu dans le composé organique.

6. Procédé de fabrication selon l'une quelconque des revendications 1 à 5, dans lequel un agent stabilisant destiné à disperser de façon stable des particules fines fabriquées dans le liquide cible de traitement est ajouté au liquide cible de traitement avant ou pendant l'irradiation par le faisceau laser du liquide cible de traitement.

7. Procédé de fabrication selon la revendication 6, dans lequel l'agent stabilisant est un agent tensioactif.

8. Procédé de fabrication selon la revendication 7, dans lequel, après que l'agent tensioactif a été ajouté au liquide cible de traitement, le liquide cible de traitement est dilué pour séparer les particules fines de l'agent tensioactif, de façon à obtenir une agrégation de particules fines qui constituent un agrégat des particules fines.

9. Procédé de fabrication selon l'une quelconque des revendications 1 à 8, dans lequel, dans l'état d'irradiation par le faisceau laser, un faisceau laser ayant une longueur d'onde prédéterminée, qui est différente de la bande d'absorption de lumière du composé organique et qui agit sur le solvant, est amené à irradier le liquide cible de traitement en tant que faisceau laser ayant la longueur d'onde plus grande que la bande d'absorption de lumière du composé organique.

10. Procédé de fabrication selon la revendication 9, dans lequel la longueur d'onde du faisceau laser est une longueur d'onde de la bande d'absorption de lumière du solvant.

11. Procédé de fabrication selon l'une quelconque des revendications 1 à 10, dans lequel la longueur d'onde du faisceau laser est de 900 nm ou plus.

12. Procédé de fabrication selon l'une quelconque des revendications 1 à 11, dans lequel le faisceau laser est amené à irradier le liquide cible de traitement en même temps que le liquide cible de traitement est refroidi.

13. Procédé de fabrication selon l'une quelconque des revendications 1 à 12, dans lequel le point de fusion du composé organique est de 250°C ou moins.

14. Appareil de fabrication de particule fine destiné à pulvériser finement le composé organique dans un solvant d'un liquide cible de traitement pour fabriquer une particule fine du composé organique, comportant :
une chambre destinée à loger un liquide cible de traitement contenant un composé organique ayant une bande d'absorption de lumière prescrite et un solvant qui sont mélangés entre eux ; et
une source de faisceau laser destinée à irradier par des faisceaux laser, ayant une longueur d'onde plus grande que la bande d'absorption de lumière du composé organique, le liquide cible de traitement logé dans la chambre,
dans lequel le composé organique est un médicament, et l'appareil de fabrication comportant en outre :
un moyen de réglage d'intensité de la lumière d'irradiation destiné à régler l'intensité de la lumière d'irradiation du faisceau laser devant irradier la chambre depuis la source de faisceau laser ; et
un moyen de commande destiné à commander le moyen de réglage de l'intensité de la lumière d'irradiation afin que l'intensité de la lumière d'irradiation du faisceau laser sur le liquide cible de traitement soit réglée de façon à être inférieure à l'intensité de lumière d'irradiation à laquelle une absorption de deux photons a lieu dans le composé organique.

15. Appareil de fabrication selon la revendication 14, dans lequel la source de faisceau laser est un laser à longueur d'onde variable.

16. Appareil de fabrication selon la revendication 15, comportant en outre un moyen de mesure de la bande d'absorption de lumière déterminant la longueur d'onde d'irradiation destiné à décharger une partie du liquide cible de traitement depuis la chambre, et à mesurer l'absorbance du composé organique dans le liquide cible de traitement afin de déterminer la longueur d'onde du faisceau laser devant irradier le composé organique ;
dans lequel le moyen de mesure de la bande d'absorption de lumière déterminant la longueur d'onde d'irradiation comporte un filtre de séparation destiné à séparer une matière solide du liquide cible de traitement déchargé de la chambre, et l'absorbance du composé organique dans le liquide cible de traitement duquel la matière solide est séparée par le filtre de séparation est mesurée.

17. Appareil de fabrication selon l'une quelconque des revendications 14 à 16, comportant en outre :
un dispositif de mesure de l'intensité de la lumière de transmission destiné à mesurer l'intensité de la lumière de transmission du faisceau laser transmis à travers le liquide cible de traitement dans la chambre.

18. Appareil de fabrication selon la revendication 17, dans lequel la chambre absorbe plus fortement un faisceau laser qui a une longueur d'onde plus grande que la bande d'absorption de lumière et qui a une intensité de lumière d'irradiation à laquelle une absorption de deux photons a lieu dans le composé organique, en comparaison avec un faisceau laser ayant une intensité de lumière d'irradiation à laquelle aucune absorption de deux photons n'a lieu.

19. Appareil de fabrication selon l'une quelconque des revendications 14 à 18, dans lequel la source de faisceau laser irradie le liquide cible de traitement logé dans la chambre avec un faisceau laser ayant une longueur d'onde prédéterminée qui est différente de la bande d'absorption de lumière du composé organique et agit sur le solvant, en tant que faisceau laser ayant une longueur d'onde plus grande que la bande d'absorption de lumière du composé organique.

20. Appareil de fabrication selon la revendication 19, dans lequel la longueur d'onde du faisceau laser est une longueur d'onde de la bande d'absorption de lumière du solvant.

21. Appareil de fabrication selon l'une quelconque des revendications 14 à 20, dans lequel la longueur d'onde du faisceau laser est établie à 900 nm ou plus.

22. Appareil de fabrication selon l'une quelconque des revendications 14 à 21, comportant en outre un moyen de mesure de bande d'absorption de lumière pour un contrôle destiné à mesurer l'absorbance du composé organique dans le liquide cible de traitement pour contrôler un état de pulvérisation fine du composé organique.

23. Médicament sous forme de particule fine fabriquée par le procédé de fabrication de particule fine selon l'une quelconque des revendications 1 à 13.

24. Procédé de fabrication d'un agent injectable, dans lequel un liquide contenant des particules fines est fabriqué par le procédé de fabrication de particules fines selon la revendication 1, et un agent d'isotonicité est ajouté au liquide pour fabriquer l'agent injectable.

25. Agent injectable fabriqué par le procédé de fabrication d'agent injectable selon la revendication 24.
